# EUROPEAN PATENT SPECIFICATION

(11) **EP 0 982 026 B1**
(45) Date of publication and mention of the grant of the patent: **17.05.2006**
(21) Application number: 99115114.3
(22) Date of filing: 09.08.1999
(51) Int. Cl.: A61K 31/137, A61K 31/18, A61K 31/357, A61K 31/4468, A61P 25/16, A61P 25/26, A61P 25/28

(54) **Use of aryl-cyclohexylamine derivatives in the manufacture of NMDA receptor blockers**
Verwendung von Aryl-Cyclohexylamine Derivaten zur Herstellung von NMDA-Rezeptorblockern
Utilisation de dérivés d'aryl-cyclohexylamine dans la fabrication d'agents bloquants du récepteur NMDA

(30) Priority: 18.08.1998 EP 98115484
(43) Date of publication of application: 01.03.2000
(73) Proprietor: F. HOFFMANN-LA ROCHE AG, 4070 Basel (CH)
(72) Inventor: Alanine, Alexander, 68400 Riedisheim (FR); Buettelmann, Bernd, 70650 Schopfheim (DE); Heitz Neidhart, Marie-Paule, 68220 Hagenthal le Bas (FR); Pinard, Emmanuel, 68480 Linsdorf (FR); Wyler, René, 8002 Zürich (CH)
(74) Representative: Poppe, Regina

(56) References cited:
- EP-A- 0 481 299
- EP-A- 0 503 411
- WO-A-97/15549
- WO-A-97/37652
- FR-A- 2 110 731
- GB-A- 1 327 691
- US-A- 3 965 180
- LYNCH D.R. ET AL THE JOURNAL OF PHARMACOLOGY AND EXPERIMENTAL THERAPEUTICS vol. 279, no. 1, 1996, pages 154 - 161

## Description

The present invention relates to the use of compounds of the general formula 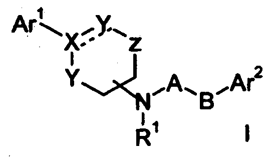 wherein
- Ar¹: is phenyl, naphthyl or tetrahydronaphthyl, optionally substituted by hydroxy, C₁-C₄ alkoxy, nitro, amino or methanesulfonamide;
- Ar²: is phenyl, naphthyl or tetrahydronaphthyl, optionally substituted by C₁-C₄ alkyl or halogen;
- X: is C, CH or C(OH);
- Y: is -CH₂-, CH or O
- Z: -CH₂-, -CH(CH₃)- or -C(CH₃)₂-;
- R¹: is hydrogen, C₁-C₄ alkyl or acetyl;
- A: is C=O or -(CHR²)ₙ-, wherein R² is hydrogen, C₁-C₄ alkyl or hydroxy-C₁-C₄ alkyl;
- B: is -(CH₂)ₙ-, O, -CH(OH)(CH₂)ₙ-, -CH(CH₂OH)(CH₂)ₙ-, -(CH₂)ₙ CH(OH)- or -CH(CH₂OH)-;
- ---: may be a bond and
- n: is 0-4
and to pharmaceutically acceptable acid addition salts thereof for the manufacture of a medicament for the treatment of neurodegeneration, caused by stroke, brain trauma, Alzheimer's disease, Parkinson's disease, Huntington's disease, ALS (amyotrophic lateral sclerosis), and neurodegeneration associated with bacterial or viral infections.

Most of the described aryl derivatives are known compounds. In EP 503 411 and EP 481 299 are described N-phenyl-4-amino-piperidines with antiarrythmic and psychotropic activities. In WO 9715549 are described compounds of the present of formula I, which have a potent effect of stimulating beta-3 adrenaline receptors. These compounds are useful for the treatment of urinary disorders such as frequent urination and urinary incontinence, convulsion and exasperation of the function of digestive tract movement, obesity and diabetes.

Furthermore, FR2110731 describes N-salicylolyl derivatives of 4-phenylcyclohexylamine possessing in particular analgetic, antipyretic, anti-inflammatory, antirheumatic, anticonvulsant and antidepressant properties.

The compounds of formula I and their salts are distinguished by valuable therapeutic properties. It has now surprisingly been found that compounds of the present invention are NMDA(N-methyl-D-aspartate)-receptor subtype selective blockers, which have a key function in modulating neuronal activity and plasticity which makes them key players in mediating processes underlying development of CNS as well as learning and memory formation.

Under pathological conditions of acute and chronic forms of neurodegeneration overactivation of NMDA receptors is a key event for triggering neuronal cell death. NMDA receptors are composed of members from two subunit families, namely NR-1 (8 different splice variants) and NR-2 (A to D) originating from different genes. Members from the two subunit families show a distinct distribution in different brain areas. Heteromeric combinations of NR-1 members with different NR-2 subunits result in NMDA receptors displaying different pharmaceutical properties. Possible therapeutic indications for NMDA receptor subtype specific blockers include acute forms of neurodegeneration caused, e.g., by stroke and brain trauma, and chronic forms of neurodegeneration such as Alzheimer's disease, Parkinson's disease, Huntington's disease, ALS (amyotrophic lateral sclerosis) and neurodegeneration associated with bacterial or viral infections.

Objects of the invention are the use of compounds of formula I and pharmaceutically acceptable acid addition salts thereof for the manufacture of medicaments for the treatment or prophylaxis of diseases, caused by overactivation of respective NMDA receptor subtype, being stroke, brain trauma, Alzheimer's disease, Parkinson's disease, Huntington's disease, ALS (amyotrophic lateral sclerosis) and neurodegeneration associated with bacterial or viral infections.

The present invention embraces racemic mixtures and all their corresponding enantiomers.

The substituation of the group -N(R¹)-A-B-Ar² may be in position 2 (in case when Y is CH₂) 3 or 4 in accordance to X.

The following definitions of the general terms used in the present description apply irrespective of whether the terms in question appear alone or in combination.

As used herein, the term "lower alkyl" denotes a straight- or branched-chain alkyl group containing from 1 to 4 carbon atoms, for example, methyl, ethyl, propyl, isopropyl, butyl and the like.

The term "halogen" denotes chlorine, iodine, fluorine and bromine.

The term "lower alkoxy" denotes a group wherein the alkyl residue is as defined above.

The term "pharmaceutically acceptable acid addition salts" embraces salts with inorganic and organic acids, such as hydrochloric acid, nitric acid, sulfuric acid, phosphoric acid, citric acid, formic acid, fumaric acid, maleic acid, acetic acid, succinic acid, tartaric acid, methane-sulfonic acid, p-toluenesulfonic acid and the like.

The term "leaving group" has the meaning conventionally used, and refers to, for example, halogen, alkylsulfonyloxy, arylsulfonyloxy and the like. The most preferred leaving group in the present case is a halogen.

The compounds of the present invention may be subdivided in the following subgroups: 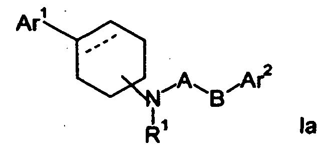 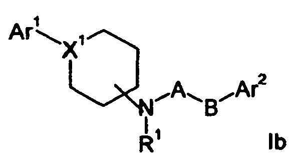 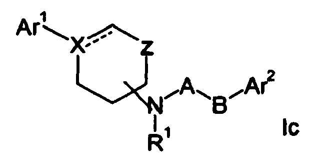 or 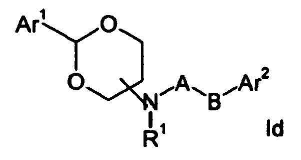 wherein Z is -CH(CH₃)- or C(CH₃)₂-, Ar¹, Ar², X, A, B, the dotted line and R¹ have the significances given above and X¹ is C(OH).

Preferred compounds of formula Ia in the scope of the present invention are the following:
trans-4- [4-(3-phenyl-propylamino)-cyclohexyl]-phenol,
trans-4-[4-[methyl-(3-phenyl-propyl)-amino]-cyclohexyl]-phenol,
trans-4-[4-[ethyl-(3-phenyl-propyl)-amino]-cyclohexyl]-phenol,
trans-4- [4-(4-phenyl-butylamino)-cyclohexyl]-phenol,
trans-4- [4- [3-(4-fluoro-phenyl)-propylamino]-cyclohexyl] phenol,
trans-4-(4-[[3-(4- fluoro-phenyl)-propyl]-methyl-amino]-cyclohexyl)-phenol,
trans-4- [4- [methyl-(2-p-tolyloxy-ethyl)-amino] -cyclohexyl]-phenol,
(RS)-4-[trans-4-(1-hydroxymethyl-3-phenyl-propylamino)-cyclohexyl]-phenol,
(RS)-4-[trans-4-(2-hydroxymethyl-3-phenyl-propylamino)-cyclohexyl]-phenol, and
trans-N-(4-[4- [methyl-(3-phenyl-propyl)-amino]-cyclohexyl]-phenyl)-methanesulfonamide.

A preferred compound of formula Ibis:
cis-4-[1-hydroxy-4-(3-phenyl-propylamino)-cydohexyl] -phenol.

A preferred compound of formula Ic is
(1RS, 3RS, 4RS)-4-[3-methyl-4- [methyl-(3-phenyl-propyl)-amino] -cyclohexyl]-phenol.

Further preferred is a compound of formula Id, which is
trans-4- [5-(3-phenyl-propylamino)-[1,3] dioxan-2-yl] -phenol.

The afore-mentioned compounds of formula I and their subgroups (Ia-Id) can be manufactured in accordance with known methods, for example by
a) reacting a compound of formula 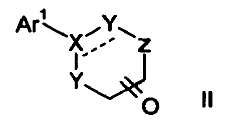 with an amine of formula 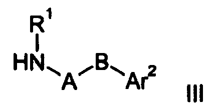 wherein Ar¹, Ar², X, Y, Z, A, B and R¹ have the significances given above, or
b) reacting a compound of formula 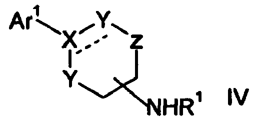 with a compound of formula 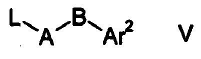 wherein L is a leaving group and Ar¹, Ar², X, Y, Z, A, B and R¹ have the significances given above, or
c) reacting a compound of formula 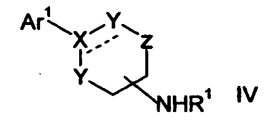 with a compound of formula 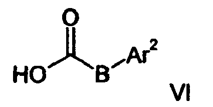 to give a compound of formula 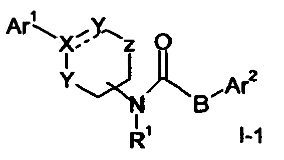 wherein Ar¹, Ar², X, Y, Z and B have the significances given above, or
d) cleaving off an O-protecting group from a compound of formula 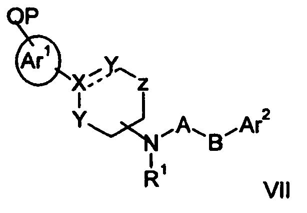
wherein Ar¹, Ar², X, Y, Z, R¹, A, B and R¹ have the significances given above and P is a protecting group, and, if desired, converting the compound of formula I obtained into a pharmaceutically acceptable salt.

Furthermore, a nitro group can be hydrogenated to an amino group, the amino group can be alkylated or converted into a group NHSO₂CH₃ by methods known in the art.

In accordance with reaction step a) a compound of formula II with an amine of formula III is heated in a Dean-Stark apparatus in a suitable solvent, such as toluene. A suitable amine is, for example, phenethylamine. The reduction of the imine intermediate is effected with a hydride donor in a suitable solvent, for example with borohydride in methanol.

In accordance with reaction variant b) a compound of formula IV is treated with a compound of formula V, wherein the most preferred leaving group is bromine. This reaction is carried out in conventional manner in the presence of K₂CO₃.

Process variant c) describes the reaction of a compound of formula IV with a corresponding acid derivative of formula VI. This reaction is carried out in the presence of CDI (carbonyl diimidazole) at 0°C for about 1 h.

In accordance with process variant d) a compound of formula VII is deprotected to a compound of general formula I. Suitable protecting groups and methods for their cleavage will be familiar to any person skilled in the art, although of course there can be used only those protecting groups which can be cleaved off by methods under conditions of which other structural elements are not affected. The benzyl group is a preferred O-protecting group. The process is carried out in conventional manner. For example, a compound of formula VII is dissolved in a suitable solvent or mixture of solvents such as methanol, and hydrogenated.

Pharmaceutically acceptable salts can be manufactured according to methods which are known per se and familiar to any person skilled in the art. The acid addition salts of compounds of formula I are especially well suited for pharmaceutical use.

In schemes 1 - 7 are described processes for preparation of compounds of formula I, starting from known compounds or from compounds, which can be prepared in conventional manner.

The starting materials of formulae II, III, VIII, IX, XII, XV, XVI, XVIII and IXX are commercial products or can be prepared according to methods known per se. The preparation of compounds of formula I are described in more detail in working examples 1 - 86. 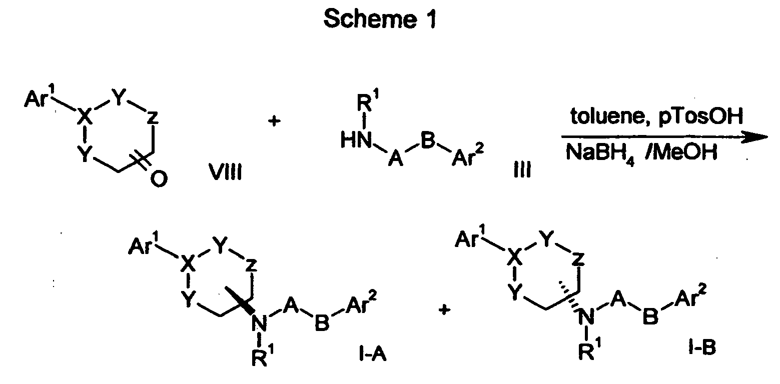 wherein the substituents are described as above. 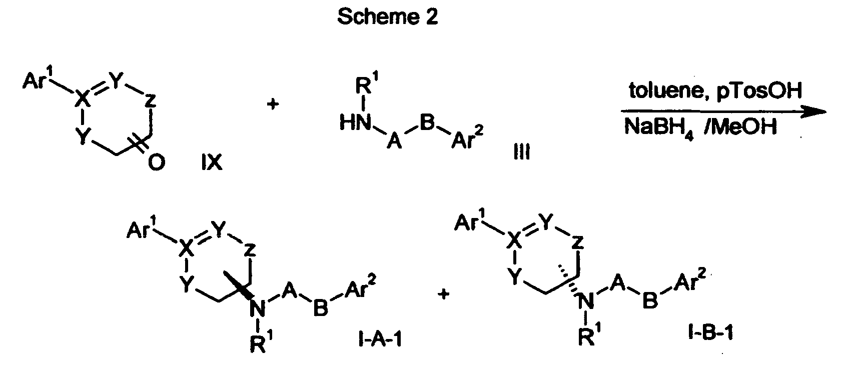 wherein the substituents are described as above. 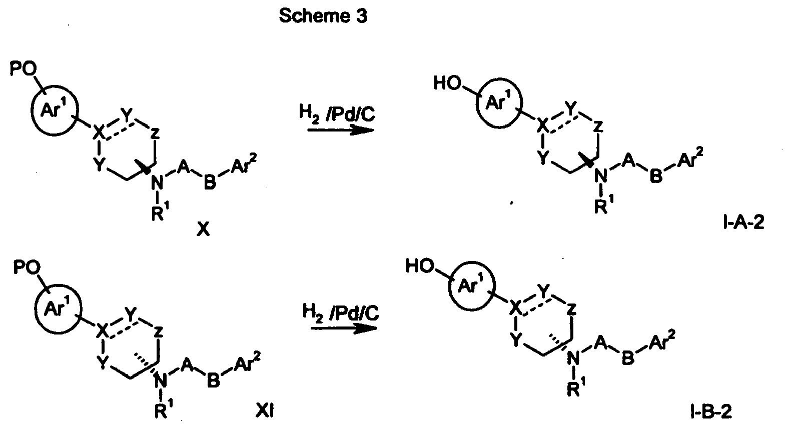 wherein the substituents are described as above. 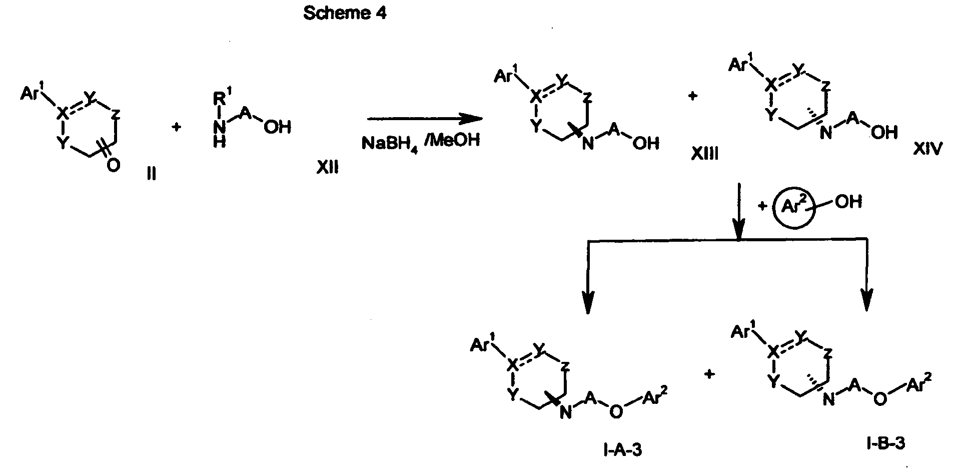 wherein the substituents are described as above. 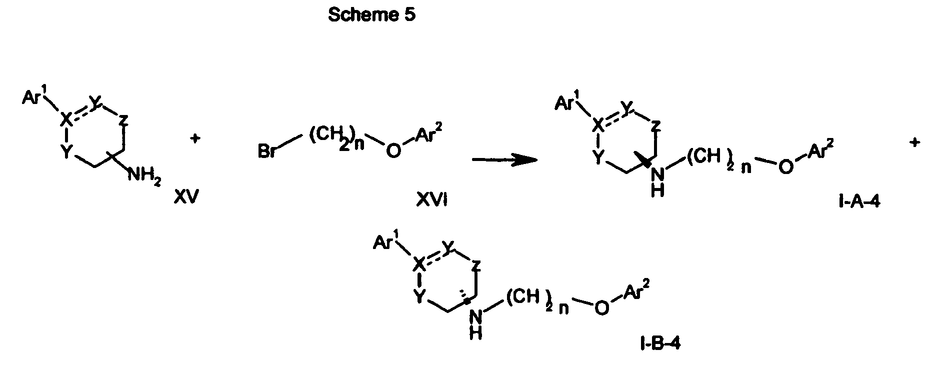 wherein the substituents are described as above. 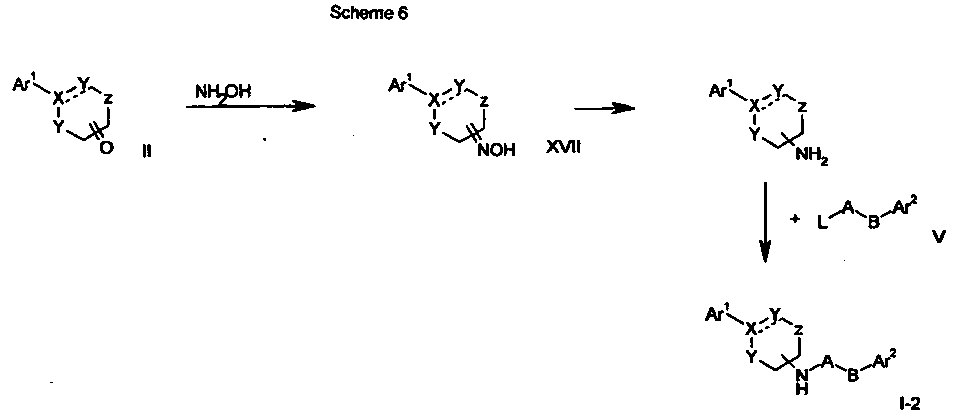 wherein the substituents are described as above. 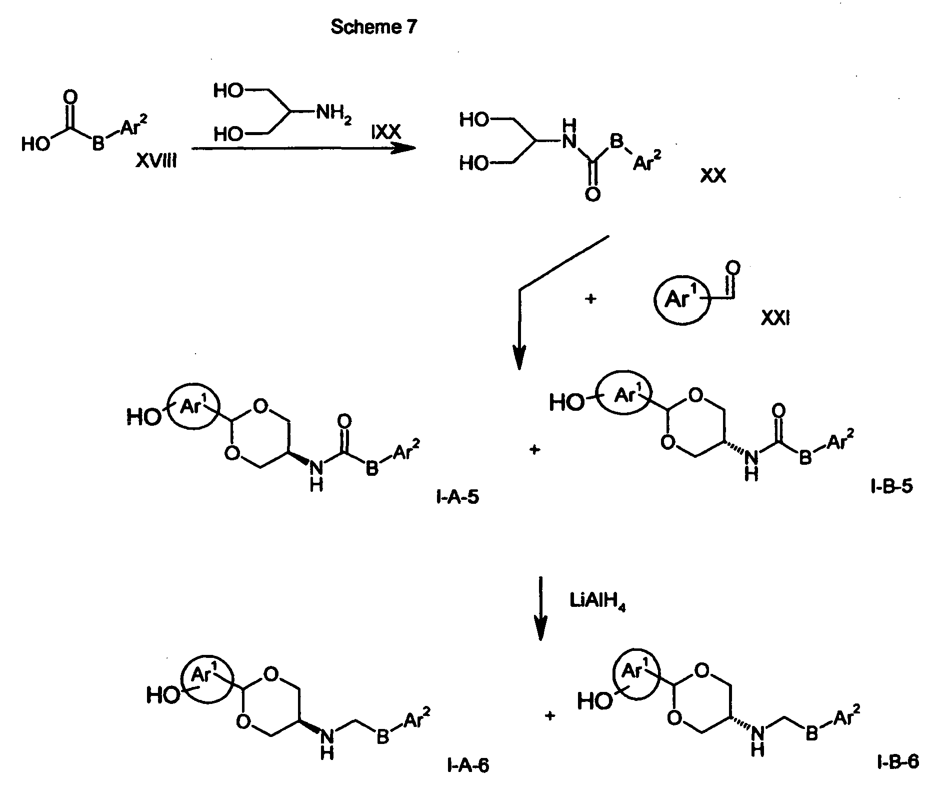 wherein the substituents are described as above.

As mentioned earlier, the compounds of formula I and their pharmaceutically usable acid addition salts possess valuable pharmacodynamic properties. They are NMDA-receptor subtype selective blockers, which have a key function in modulating neuronal activity and plasticity which makes them key players in mediating processes underlying development of CNS as well as learning and memory formation.

The compounds were investigated in accordance with the test given ereinafter.

### Test method

### 3H-Ro 25-6981 binding (Ro 25-6981 is [R-(R*,S*)]-a-(4-Hydroxy-phenyl)-b-methyl-4-(phenyl-methyl)-1-piperidine propanol)

Male Füllinsdorf albino rats weighing between 150-200 g were used. Membranes were prepared by homogenization of the whole brain minus cerebellum and medulla oblongata with a Polytron (10.000 rpm, 30 seconds), in 25 volumes of a cold Tris-HCl 50 mM, EDTA 10 mM, pH 7.1 buffer. The homogenate was centrifuged at 48.000 g for 10 minutes at 4°C. The pellet was resuspended using the Polytron in the same volume of buffer and the homogenate was incubated at 37°C for 10 minutes. After centrifugation the pellet was homogenized in the same buffer and frozen at -80°C for at least 16 hours but not more than 10 days. For the binding assay the homogenate was thawed at 37°C, centrifuged and the pellet was washed three times as above in a Tris-HCl 5 mM, pH 7.4 cold buffer. The final pellet was resuspended in the same buffer and used at a final concentration of 200 mg of protein/ml.

3H-Ro 25-6981 binding experiments were performed using a Tris-HCl 50 mM, pH 7.4 buffer. For displacement experiments 5 nM of 3H-Ro 25-6981 were used and non specific binding was measured using 10 mM of tetrahydroisoquinoline and usually it accounts for 10% of the total. The incubation time was 2 hours at 4°C and the assay was stopped by filtration on Whatmann GF/B glass fiber filters (Unifilter-96, Packard, Zürich, Switzerland). The filters were washed 5 times with cold buffer. The radioactivity on the filter was counted on a Packard Top-count microplate scintillation counter after addition of 40 mL of microscint 40 (Canberra Packard S.A., Zürich, Switzerland).

The effects of compounds were measured using a minimum of 8 concentrations and repeated at least once. The pooled normalized values were analyzed using a non-linear regression calculation program which provide IC₅₀ with their relative upper and lower 95% confidence limits (RS1, BBN, USA).

The IC₅₀(µM) of preferred compounds tested in accordance with the above mentioned methods are in the range of about 0.004 - 0.01.

In the table below some data of active compouds are given:

| Example No. | IC₅₀ in µM |
|---|---|
| 22b | 0.02 |
| 24 | 0.07 |
| 39b | 0.009 |
| 40c | 0.04 |
| 41b | 0.02 |
| 43b | 0.02 |
| 44b | 0.008 |
| 45b | 0.13 |
| 46b | 0.01 |
| 47b | 0.03 |
| 48c | 0.04 |
| 50 | 0.08 |
| 56 | 0.011 |
| 57b | 0.01 |
| 58 | 0.04 |
| 60b | 0.007 |
| 61b | 0.004 |
| 62b | 0.006 |
| 63b | 0.02 |
| 64b | 0.06 |
| 65b | 0.01 |
| 66c | 0.02 |
| 67b | 0.1 |
| 70b | 0.1 |
| 71c | 0.025 |
| 73b | 0.01 |
| 74c | 0.01 |
| 75 | 0.022 |

The compounds of formula I and their salts, as herein described, can be incorporated into standard pharmaceutical dosage forms, for example, for oral or parenteral application with the usual pharmaceutical adjuvant materials, for example, organic or inorganic inert carrier materials, such as, water, gelatin, lactose, starch, magnesium stearate, talc, vegetable oils, gums, polyalkylene-glycols and the like. The pharmaceutical preparations can be employed in a solid form, for example, as tablets, suppositories, capsules, or in liquid form, for example, as solutions, suspensions or emulsions. Pharmaceutical adjuvant materials can be added and include preservatives stabilizers, wetting or emulsifying agents, salts to change the osmotic pressure or to act as buffers. The pharmaceutical preparations can also contain other therapeutically active substances.

The dosage can vary within wide limits and will, of course, be fitted to the individual requirements in each particular case. In the case of oral administration the dosage lies in the range of 0.1 mg per dosage to 1000 mg per day of a compound of general formula I although the upper limit can also be exceeded when this is shown to be indicated.

The following Examples illustrate the present invention in more detail. A11 temperatures are given in degrees celsius.

### Example 1

### (1RS,2RS)-4-(2-Phenthylamino-cyclohexyl)-phenol

A 1M BBr₃-CH₂Cl₂ solution (9.4 ml, 9.4 mmol) was added to a solution of cis-[2-(4-methoxy-phenyl)-cyclohexyl]-phenethyl-amine (1.45 g, 4.69 mmol) in CH₂Cl₂ at 0°. After stirring for 2 h, the reaction mixture was poured on ice, treated with sat. NaHCO₃ solution and extracted with CH₂Cl₂. The residue was purified by chromatography over SiO₂ (Merck 230-400 mesh) eluting with CH₂Cl₂-MeOH-25% aq. NH₃ 140:10:1 to give after trituration with ether-hexane 570 mg of (1RS,2RS)-4-(2-phenethylamino-cyclohexyl)-phenol as a light brown powder. Mp. 121°, MS: m/e=296.3 (M+H⁺).

### Example 2

### (1RS,2SR)-4-(2-Phenethylamino-cyclohexyl)-phenol

Following the general method of example 1, (1RS,2SR)-[2-(4-methoxy-phenyl)-cyclohexyl]-phenethyl-amine (0.9 g, 2.9 mmol) was converted to (1RS,2SR)-4-(2-phenethylamino-cyclohexyl)-phenol, giving 0.63 g of a light brown powder. MS: m/e=296.3 (M+H⁺).

### Example 3

### (1RS,2RS)-[2-(4-Methoxy-phenyl)-cyclohexyl]-phenethyl-amine and (1RS,2SR)-[2-(4-Methoxy-phenyl)-cyclohexyl]-phenethyl-amine

A mixture of 2-(4-methoxyphenyl)-cyclohexanone (prepared following the procedure described in W. E. Bachmann et al., J. Am. Chem. Soc., 1950, 72, 1995) (2.0 g, 9.79 mmol), phenethylamine (1.19 g, 9.79 mmol), toluene (40 ml) and a catalytical amount of p-toluenesulfonic acid was refluxed in a Dean-Stark apparatus overnight. After removal of the solvent, the residue was dissolved in MeOH (25 ml) and sodium borohydride (excess) was added. After stirring for 1 h at RT, the solvent was evaporated, H₂O was added to the residue. Extraction with CH₂Cl₂, drying of the organic layer over Na₂SO₄, evaporation of the solvent and chromatography of the residue over SiO₂ (Merck 230-400 mesh) eluting with AcOEt gave (1RS,2RS)-[2-(4-methoxy-phenyl)-cyclohexyl]-phenethyl-amine (1.5 g, 50%, light yellow oil, MS: m/e=310.2 (M+H⁺)) and (1RS,2SR)-[2-(4-methoxy-phenyl)-cyclohexyl]-phenethyl-amine (0.95 g, 31%, light yellow oil, MS: m/e=310.2 (M+H⁺)).

### Example 4

### (1RS,2RS)-4- [2-(3-Phenyl-propylamino)-cydohexyl]-phenol

Following the general method of example 1 (2RS,3RS)-[2-(4-methoxy-phenyl)-cyclohexyl]-(3-phenyl-propyl)-amine (0.4 g, 1.24 mmol) was converted to (1RS,2RS)-4-[2-(3-phenyl-propylamino)-cyclohexyl]-phenol, giving 0.27 g of a light yellow oil. MS: m/e=310.3 (M+H⁺).

### Example 5

### (1RS,2SR)-4-[2-(3-Phenyl-propylamino)-cyclohexyl]-phenol

Following the general method of example 1 (2RS,3SR)-[2-(4-methoxy-phenyl)-cyclohexyl]-(3-phenyl-propyl)-amine (0.28 g, 0.87 mmol) was converted to (1RS,2SR)-4-[2-(3-phenyl-propylamino)-cyclohexyl]-phenol, giving 0.17 g of a light brown solid. MS: m/e=310.3 (M+H⁺).

### Example 6

### (2RS.3RS)-[2-(4-Methoxy-phenyl)-cycloxyl]-(3-phenyl-propyl)-amine and (2RS,3SR)-[2-(4-Methoxy-phenyl)-cyclohexyl]-(3-phenyl-propyl)-amine

Following the general method of example 3 (2RS,3RS)-[2-(4-methoxy-phenyl)-cyclohexyl]-(3-phenyl-propyl)-amine (510 mg; 16%), colorless oil, MS: m/e=324.4 (M+H⁺)) and (2RS,3SR)-[2-(4-methoxy-phenyl)-cyclohexyl]-(3-phenyl-propyl)-amine (310 mg; 10%) colorless oil, MS: m/e=324.4 (M+H⁺)) were prepared from 2-(4-methoxyphenyl)-cyclohexanone (2.0 g; 9.8 mmol) and 3-phenylpropylamine (1.32 g; 9.8 mmol).

### Example 7

### (1RS,2RS)-4-[2-(4-Phenyl-butylamino)-cyclohexyl]-phenol

Following the general method of example 1 (2RS,3RS)-[2-(4-methoxy-phenyl)-cyclohexyl]-(3-phenyl-butyl)-amine (1.75 g, 5.19 mmol) was converted to (1RS,2RS)-4-[2-(3-phenyl-butylamino)-cyclohexyl]-phenol, giving 0.50 g of a white solid. MS: m/e=324.3 (M+H⁺).

### Example 8

### (1RS.2SR)-4- [2-(4-Phenyl-butylamino)-cyclohexyl]-phenol

Following the general method of example 1 (2RS,3SR)-[2-(4-methoxy-phenyl)-cyclohexyl]-(3-phenyl-butyl)-amine (0.95 g, 2.81 mmol) was converted to (1RS,2SR)-4-[2-(3-phenyl-butylamino)-cyclohexyl]-phenol, giving 0.45 g of a light brown solid. MS: m/e=324.3 (M+H⁺).

### Example 9

### (1RS,2RS)-[2-(4-Methoxy-phenyl)-cyclohexyl]-(4-phenyl-butyl)-amine and (1RS,2SR) f 2-(4-Methoxy-phenyl)-cyclohexyl]-(4-phenyl-butyl-amine

Following the general method of example 3 (2RS,3RS)-[2-(4-methoxy-phenyl)-cyclohexyl]-(3-phenyl-butyl)-amine (1.8 g; 54%) light yellow oil, MS: m/e=338.3 (M+H⁺)) and (2RS,3SR)-[2-(4-methoxy-phenyl)-cyclohexyl]-(3-phenyl-butyl)-amine (1.0 g; 30% light yellow oil, MS: m/e=338.3 (M+H⁺)) were prepared from 2-(4-methoxyphenyl)-cyclohexanone(2.0 g; 9.8 mmol) and 4-phenylbutylamine (1.46 g; 9.8 mmol).

### Example 10

### (1RS,2RS)-3-(2-Phenethylamino-cyclohexyl)-phenol

(1RS,2RS)-[2-(3-methoxy-phenyl)-cyclohexyl]-phenethyl-amine hydrochloride (1:1) (0.7 g, 2.26 mmol) was first treated with NaHCO₃ and then, following the general method of example 1, was converted to (1RS,2RS)-3-(2-phenethylamino-cyclohexyl)-phenol, giving 0.51 g of a light brown powder. MS: m/e=296.3 (M+H⁺).

### Example 11

### (1RS,2SR)-3-(2-Phenethylamino-cyclohexyl)-phenol

Following the general method of example 1, (1RS,2SR)-[2-(3-methoxy-phenyl)-cyclohexyl]-phenethyl-amine (1.7 g, 5.49 mmol) was converted to (1RS,2SR)-3-(2-phenethylamino-cyclohexyl)-phenol, giving 1.04 g of white crystalline material. Mp. 164-165°, MS: m/e=296.3 (M+H⁺).

### Example 12

### (1RS,2RS)-[2-(3-Methoxy-phepyl)-cyclohexyl]-phenethyl-amine hydrochloride (1:1) and (1RS,2SR)-[2-(3-Methoxy-phenyl)-cyclohexyl]-phenethyl-amine

Following the general method of example 3 (2RS,3RS)-[2-(4-methoxy-phenyl)-cyclohexyl]-phenetyl-amine (of which the hydrochloride salt was prepared with HCl in Ether: 1.3 g; 15% light brown solid, MS: m/e=310.2 (M+H⁺)) and (2RS,3SR)-[2-(4-methoxy-phenyl)-cyclohexyl]-phenetyl-amine (1.7 g; 22% light yellow oil, MS: m/e=310.2 (M+H⁺)) were prepared from 2-(3-methoxyphenyl)-cyclohexanone (5.0 g; 24.5 mmol) and phenethylamine (2.97 g; 24.5 mmol).

### Example 13

### (1RS,2RS)-3-[2-(3-Phenyl-propylamino)-cyclohexyl]-phenol

Following the general method of example 1, (1RS,2RS)-[2-(3-methoxy-phenyl)-cyclohexyl]-(3-phenyl-propyl)-amine (1.6 g, 4.95 mmol) was converted to (1RS,2RS)-3-[2-(3-phenyl-propylamino)-cyclohexyl]-phenol, giving 0.85 g of a white crystalline material. Mp. 112-113°, MS: m/e=310.2 (M+H⁺).

### Example 14

### (1RS,2SR)-3-[2-(3-Phenyl-propylamino)-cyclohexyl]-phenol

Following the general method of example 1, (1RS,2SR)-[2-(3-methoxy-phenyl)-cyclohexyl]-(3-phenyl-propyl)-amine (1.2 g, 3.71 mmol) was converted to (1RS,2SR)-3-[2-(3-phenyl-propylamino)-cyclohexyl]-phenol, giving 0.65 g of a white crystalline material. Mp. 112-113°, MS: m/e=310.2 (M+H⁺).

### Example 15

### (1RS,2RS)-[2-(3-Methoxy-Methoxy-phenyl)-cyclohexyl]-(3-phenyl-propyl)-amine and (1RS,2SR)-[2-(3-Methoxy-phenyl)-cyclohexyl]-(3-phenyl-propyl)-amine

Following the general method of example 3 (2RS,3RS)-[2-(3-methoxy-phenyl)-cyclohexyl]-(3-phenyl-propyl)-amine (1.63 g; 21% light yellow oil, MS: m/e=324.4 (M+H⁺)) and (2RS,3SR)-[2-(3-methoxy-phenyl)-ryclohexyl]-(3-phenyl-propyl)-amine (1.28 g; 16% light yellow oil, MS: m/e=324.4 (M+H⁺)) were prepared from 2-(3-methoxyphenyl)-cyclohexanone (5.0 g; 24.5 mmol) and 3-phenylpropylamine(3.31 g; 24.5 mmol).

### Example 16

### (1RS,2RS)-3-[2-(4-Phenyl-butylamino)cyclohexyl]-phenol

Following the general method of example 1, (1RS,2RS)-[2-(3-methoxy-phenyl)-cyclohexyl]-(4-phenyl-butyl)-amine (1.6 g, 4.95 mmol) was converted to (1RS,2RS)-3-[2-(4-phenyl-butylamino)-cyclohexyl]-phenol, giving 0.64 g of a light brown crystalline material. Mp. 115-116°, MS: m/e=324.3 (M+H⁺).

### Example 17

### (1RS,2SR)-3- [2-(4-Phenyl-butylamino)-cydohgxyl]-phenol

Following the general method of example 1, (1RS,2SR)-[2-(3-methoxy-phenyl)-cyclohexyl]-(4-phenyl-butyl)-amine (1.6 g, 4.95 mmol) was converted to (1RS,2SR)-3-[2-(4-phenyl-butylamino)-cyclohexyl]-phenol, giving 0.98 g of a light brown crystalline material. Mp. 131-132°, MS: m/e=324.3 (M+H⁺).

### Example 18

### (1RS,2RS)-[2-(3-Methoxy-phenyl)-cyclohexyl]-(4-phenyl-butyl)-amine hydrochloride (1:1) and (1RS,2SR)-[2-(3-Methoxy-phenyl)-cyclohexyl]-(4-phenyl-butyl)-amine

Following the general method of example 3 (2RS,3RS)-[2-(3-methoxy-phenyl)-cyclohexyl]-(4-phenyl-butyl)-amine (of which the hydrochloride salt was prepared with HCl in Ether, 1.8 g; 20% light brown solid, MS: m/e=338.2 (M+H⁺)) and (2RS,3SR)-[2-(3-methoxy-phenyl)-cyclohexyl]-(4-phenyl-butyl)-amine (1.6 g; 19% light yellow oil, MS: m/e=338.2 (M+H⁺)) were prepared from 2-(3-methoxyphenyl)-cyclohexanone(5.0 g; 24.5 mmol) and 4-phenylbutylamine (3.65 g; 24.5 mmol).

### Example 19

### cis-4-(4-Phenethylamino-cyclohexyl)-phenol

Following the general method of example 22b, cis-[4-(4-benzyloxy-phenyl)-cyclohexyl]-phenethyl-amine (140 mg) was hydrogenated to give cis-4-(4-phenethylamino-cyclohexyl)-phenol as white crystals (52 mg; 49 %). MS: m/e=296.4 (M+H⁺).

### Example 20

### trans-4-(4-Phenethylamino-cyclohexyl)-phenol

### a) cis-[4-(4-Benzyloxy-phenyl)-cyclohexyl]-phenethyl-amine and trans-[4-(4-Benzyloxyphenyl)-cyclohexyl]-phenethyl-amine

Following the general method of example 3, cis- [4-(4-benzyloxy-phenyl)-cyclohexyl]-phenethyl-amine, (260 mg; 11% light yellow oil, MS: m/e=386.2 (M+H⁺)) trans-[4-(4-benzyloxy-phenyl)-cyclohexyl]-phenethyl-amine (370 mg; 15 % white crystals, MS: m/e=386.3 (M+H⁺)) were prepared from 4-(4-benzyloxy-phenyl)-cyclohexanone (1.8 g; 6.4 mmol) and phenetylamine (0.78 g; 6.4 mmol).

### b) trans-4-(4-Phenethylamino-cyclohexyl)-phenol

Following the general method of 22b, trans-[4-(4-benzyloxy-phenyl)-cyclohexyl]-phenethyl-amine (190 mg) was hydrogenated to give trans-4-(4-phenethylamino-cyclohexyl)-phenol as white crystals (125 mg; 86%). MS: m/e=296.4 (M+H⁺).

### Example 21

### cis-4-[4-(3-Phenyl-propylamino)-cyclohexyl]-phenol hydrochloride (1:1)

Following the general method of 22b, cis-[4-(4-benzyloxy-phenyl)-cyclohexyl]-(3-phenylpropyl)-amine (200 mg; 0.5 mmol) was hydrogenated to give cis-4-[4-(3-phenyl-propylamino)-cyclohexyl]-phenol. The hydrochloride salt was prepared with HCl in ether to give white crystals (145 mg; 84 %). MS: m/e=310.2 (M+H⁺).

### Example 22

### trans-4-[4-(3-Phenyl-propylamino)-gyclohexyl-phenol

### a) cis-[4-(4-Benzyloxy-phenyl)-cylohexyl]-(3-(3-phenyl-propyl)-amine and trans-[4-(4-Benzyloxy-phenyl)-cyclohexyl]-(3-phenyl-propyl)-amine

Following the general method of example 3, cis-[4-(4-benzyloxy-phenyl)-cyclohexyl]- (3-phenyl-propyl)-amine, (710 mg; 17% light yellow oil, MS: m/e=400.4 (M+H⁺)) trans-[4-(4-benzyloxy-phenyl)-cyclohexyl]- (3-phenyl-propyl)-amine (1.26 g; 30 % white crystals, MS: m/e=400.4 (M+H⁺)) were prepared from 4-(4-benzyloxy-phenyl)-cyclohexanone (3.0 g; 10.7 mmol) and 3-phenyl-propylamine(1.45 g; 10.7 mmol).

### b) trans-4-[4-(3-Phenyl-propylamino)-cpclohexyl-phenol

A mixture of trans-[4-(4-benzyloxy-phenyl)-cyclohexyl]-(3-phenyl-propyl)-amine (200 mg, 0.5 mmol), Pd/C 10% (20mg) and MeOH (20 ml) was hydrogenated at RT for 3 h. Removal of the catalyst and evaporation of the solvent left a residue which after trituration with ether gave trans-4-[4-(3-phenyl-propylamino)-cyclohexyl]-phenol (130 mg, 84%) as a white crystalline material. MS: m/e=310.3 (M+H⁺).

### Example 23

### cis-4-[4-(4-Phenyl-butylamino)-cyclohexyl-phenol hydrochloride (1:1)

### a) cis-[4-(4-Benzyloxy-phenyl)-cyclohexyl]-(4-phenyl-butyl)-amine and trans-[4-(4-Benzyloxy-phenyl)-cydohexyl]-(4-phenyl-butyl)-amine

Following the general method of example 3, cis-[4-(4-benzyloxy-phenyl)-cyclohexyl]-(4-phenyl-butyl)-amine, (780 mg; 18 % light yellow oil, MS: m/e=414.4 (M+H⁺)) trans-[4-(4-benzyloxy-phenyl)-cyclohexyl]- (4-phenyl-butpyl)-amine (1.25 g; 28 % white crystals, MS: m/e=414.4 (M+H⁺)) were prepared from 4-(4-benzyloxy-phenyl)-cyclohexanone (3.0 g; 10.7 mmol) and 4-phenyl-butylamine (1.6 g; 10.7 mmol).

### b) cis-4- [4-(4-Phenyl-butylaminol-cyclohexyl]-phenol hydrochloride (1:1)

Following the general method of example 22b, cis-[4-(4-benzyloxy-phenyl)-cyclohexyl]-(4-phenyl-butyl)-amine (200 mg; 0.48 mmol) was hydrogenated to give cis-4-[4-(4-phenyl-butylamino)-cyclohexyl]-phenol. The hydrochloride salt was prepared with HCl in ether to give white crystals (140 mg; 80 %). MS: m/e=324.4 (M+H⁺).

### Example 24

### trans-4- [4-(4-Phenyl-butylamino)-cyclohexyl]-phenol

Following the general method of example 22b, trans-[4-(4-benzyloxy-phenyl)-cyclohexyl]-(4-phenyl-butyl)-amine (200 mg) was hydrogenated to give trans-4-[4-(4-phenyl-butylamino)-cyclohexyl]-phenol as white crystals (150 mg; 96 %). MS: m/e=324.3 (M+H⁺).

### Example 25

### (1RS,3SR)-4-(3-Phenethylamino-cyclohexyl)-phenol

Following the general method of example 1, (1RS,3SR)-[3-(4-methoxy-phenyl)-cyclohexyl]-phenethyl-amine (0.565 g, 1.83 mmol) was converted to (1RS,3SR)-4-(3-phenethylamino-cyclohexyl)-phenol (0.15 g, 28%, white solid, MS: m/e=296.4 (M+H⁺)).

### Example 26

### (1RS,3RS)-4-(3-Phenethylamino-cyclohexyl)-phenol

Following the general method of example 1, (1RS,3RS)-[3-(4-methoxy-phenyl)-cyclohexyl]-phenethyl-amine (0.23 g, 0.74 mmol) was converted to (1RS,3RS)-4-(3-phenethylamino-cyclohexyl)-phenol (0.13 g, 59%, light yellow solid, MS: m/e=296.4 (M+H⁺)).

### Example 27

### (1RS,3SR)-[3-(4-Methoxy-phenyl)-cyclohexyl]-phenethyl-amine and (1RS,3RS)-[3-(4-Methoxy-phenyl)-cyclohexyl]-phenethyl-amine

A mixture of (RS)-[3-(4-methoxy-phenyl)-cyclohex-2-enyl]-phenethyl-amine (1.5 g, 4.88 mmol), 10% Pd/C (0.3 g) and MeOH (30 ml) was hydrogenated for 3 h. Removal of the catalyst, evaporation of the solvent and separation of the isomers by flash-chromatography over SiO₂ (Biotage 40, 90 g) eluting with AcOEt gave (1RS,3SR)-[3-(4-methoxy-phenyl)-cyclohexyl]-phenethyl-amine (0.57 g, 38%, light yellow oil, MS: m/e=310.2 (M+H⁺)) and (1RS,3RS)-[3-(4-methoxy-phenyl)-cyclohexyl]-phenethyl-amine (0.25 g, 17%, light yellow oil, MS: m/e=310.2 (M+H⁺)).

### Example 28

### (RS)-[3-(4-MethgU-Rhenyl)-gyclohex-2-enyll -Rhenethyl-amine

A mixture of 3-(4-methoxy-phenyl)-cyclohex-2-enone (prepared following the procedure described in CA:54-10947d) (2.0 g, 9.89 mmol), phenethylamine (1.20 g, 9.89 mmol), toluene (50 ml) and a catalytical amount of p-toluenesulfonic acid was refluxed in a Dean-Stark apparatus overnight. After removal of the solvent, the residue was dissolved in MeOH (30 ml) and sodium borohydride (excess) was added. After stirring for 1 h at RT, the solvent was evaporated, H₂O was added to the residue. Extraction with CH₂Cl₂, drying of the organic layer over Na₂SO₄, evaporation of the solvent and flash-chromatography of the residue over SiO₂ (Biotage-40, 90 g) eluting with CH₂Cl₂-MeOH 95:5 gave (RS)-[3-(4-methoxy-phenyl)-cyclohex-2-enyl]-phenethyl-amine (1.7 g, 56%, light yellow solid, MS: m/e=308.2 (M+H⁺)).

### Example 29

### (RS)-4-(3-Phenethylamino-cyclohex-1-enyl)phenol

Following the general method of example 1 (RS)-[3-(4-methoxy-phenyl)-cyclohex-2-enyl]-phenethyl-amine (0.15 g, 0.49 mmol) was converted to (RS)-4-(3-phenethylamino-cyclohex-1-enyl)-phenol, giving 0.04 g of a light yellow solid. MS: m/e=294.3 (M+H⁺).

### Example 30

### (1RS,3SR)-4-[3-(3-Phenyl-propylamino)-cyclohexyl]-phenol

Following the general method of example 1, (1RS,3SR)-[3-(4-methoxy-phenyl)-cyclohexyl]-(3-phenyl-propyl)-amine (0.635 g, 1.96 mmol) was converted to (1RS,3SR)-4-[3-(3-phenyl-propylamino)-cyclohexyl]-phenol (0.3 g, 49%, light yellow oil, MS: m/e=310.2 (M+H⁺)).

### Example 31

### (1RS,3RS)-4-[3-(3-Phenyl-propylamino)-cyclohexyl-phenol

Following the general method of example 1, (1RS,3RS)-[3-(4-methoxy-phenyl)-cyclohexyl]-(3-phenyl-propyl)-amine (0.31 g, 0.96 mmol) was converted to (1RS,3RS)-4-[3-(3-phenyl-propylamino)-cyclohexyl]-phenol (0.15 g, 51%, light yellow oil, MS: m/e=310.3 (M+H⁺)).

### Example 32

### (1RS,3SR)-[3-(4-Methoxy-phenyl)-cyclohexyl]-(3-phenyl-propyl)-amine and (1RS,3RS)-[3-(4-Methoxy-phenyl)-cyclohexyl]-(3-phenyl-propyl)-amine

A mixture of (RS)-[3-(4-methoxy-phenyl)-cyclohex-2-enyl]-(3-phenyl-propyl)-amine (1.54 g, 4.79 mmol), 10% Pd/C (0.3 g) and MeOH (30 ml) was hydrogenated for 3 h. Removal of the catalyst, evaporation of the solvent and separation of the isomers by flash-chromatography over SiO₂ (Biotage 40, 90 g) eluting with AcOEt gave (1RS,3SR)-[3-(4-methoxy-phenyl)-cyclohexyl]-(3-phenyl-propyl)-amine (0.64 g, 41%, light yellow oil, MS: m/e=324.4 (M+H⁺)) and (1RS,3RS)-[3-(4-methoxy-phenyl)-cyclohexyl]-(3-phenylpropyl)-amine (0.34 g, 22%, light yellow oil, MS: m/e=324.4 (M+H⁺)).

### Example 33

### (RS)-[3-(4-Methoxy-phenyl)-cyclohex-2-enyl]-(3-phenyl-)-amine

Following the general procedure of example 28, (RS)-[3-(4-methoxy-phenyl)-cyclohex-2-enyl]-(3-phenyl-propyl)-amine (1.9 g; 40 % light yellow oil, MS: m/e=322.3 (M+H⁺)) was prepared from 3-(4-methoxy-phenyl)-cyclohex-2-enone (3.0 g; 14.8 mmol) and 3-phenyl-propylamine (2.0 g; 14.8 mmol).

### Example 34

### (RS)-4-[3-(3-Phenyl-propylamino)-cyclohex-1-enyl]-phenol

Following the general method of example 1 (RS)-[3-(4-methoxy-phenyl)-cyclohex-2-enyl]-(3-phenyl-propyl)-amine (0.20 g, 0.62 mmol) was converted to (RS)-4-[3-(3-phenyl-propylamino)-cyclohex-1-enyl]-phenol, giving 0.06 g of a light yellow oil. MS: m/e=308.3 (M+H⁺).

### Example 35

### (1RS,3SR)-4-[3-(4-Phenyl-butylamino)-cydohexyl]-phenol

Following the general method of example 1, (1RS,3SR)-[3-(4-methoxy-phenyl)-cyclohexyl]-(4-phenyl-butyl)-amine (1.1 g, 3.26 mmol) was converted to (1RS,3SR)-4-[3-(4-phenyl-butylamino)-cyclohexyl]-phenol (0.7 g, 66%, light yellow oil, MS: m/e=324.4 (M+H⁺)).

### Example 36

### (1RS,3RS)-4-[3-(4-Phenyl-butylamino)-cyclohexyl]-phenol

Following the general method of example 1, (1RS,3RS)-[3-(4-methoxy-phenyl)-cyclohexyl]-(4-phenyl-butyl)-amine (0.4 g, 1.19 mmol) was converted to (1RS,3RS)-4-[3-(4-phenyl-butylamino)-cyclohexyl]-phenol (0.16 g, 42%, light yellow oil, MS: m/e=324.4 (M+H⁺)).

### Example 37

### (1RS,3SR)-[3-(4-Methoxy-phenyl)-cyclohexyl]-(4-phenyl-butyl)-amine and (1RS,3RS)-[3-(4-Methoxy-phenl)-cyclohexyl-(4-phexyl-butyl)-amine

A mixture of (RS)-[3-(4-methoxy-phenyl)-cyclohex-2-enyl]-(4-phenyl-butyl)-amine (2.4 g, 7.15 mmol), 10% Pd/C (0.48 g) and MeOH (50 ml) was hydrogenated for 3 h. Removal of the catalyst, evaporation of the solvent and separation of the isomers by flash-chromatography over SiO₂ (Biotage 40, 90 g) eluting with AcOEt gave (1RS,3SR)-[3-(4-methoxy-phenyl)-cyclohexyl]-(4-phenyl-butyl)-amine (1.2 g, 50%, light yellow oil, MS: m/e=338.3 (M+H+)) and (1RS,3RS)-[3-(4-methoxy-phenyl)-cyclohexyl]-(4-phenylbutyl)-amine (0.43 g, 18%, light yellow oil, MS: m/e=338.3 (M+H⁺)).

### Example 38

### (RS)-[3-(4-Methoxy-phenyl)-cyclohex-2-enyl]-(4-phenyl-butyl)-amine

Following the general procedure of example 28, (RS)-[3-(4-methoxy-phenyl)-cyclohex-2-enyl]-(3-phenyl-butyl)-amine (2.8 g; 56 % light yellow oil, MS: m/e=336.2 (M+H⁺)) was prepared from 3-(4-methoxy-phenyl)-cyclohex-2-enone (3.0 g; 14.8 mmol) and 4-phenyl-butylamine (2.21 g; 14.8 mmol).

### Example 39

### trans-4-[4-[Methyl-(3-phenyl-propyl)-amino]-cyclohexyl]-phenol

### a) trans-[4-(4-Benzyloxy-phenyl)-circlohexyl]-methyl-(3-phenyl-propyl)-amine

A mixture of trans-[4-(4-benzyloxy-phenyl)-cyclohexyl]-(3-phenyl-propyl)-amine (0.4 g, 1.0 mmol), 36.5% aq. formaldehyde (0.12 ml) and MeOH (7 ml) was stirred overnight at RT. Excess sodium borohydride was then added, and stirring was continued for 2 h. Evaporation of the solvent, addition of H₂O, extraction with CH₂Cl₂, drying of the organic layer with Na₂SO₄, evaporation of the solvent and purification of the residue by chromatography over SiO₂ (Merck 230-400 mesh) eluting with CH₂Cl₂-MeOH-25% aq. NH₃ 140:10:1 trans-[4-(4-benzyloxy-phenyl)-cyclohexyl]-methyl-(3-phenyl-propyl)-amine (0.41 g, 99%) as a colorless oil. MS: m/e=413 (M⁺).

### b) trans-4-{4-[Methyl-(3-phenyl-propyl)-amino]-cyclohexyl}-phenol

Following the general method of example 22b, trans-[4-(4-benzyloxy-phenyl)-cyclohexyl]-methyl-(3-phenyl-propyl)-amine (400 mg; 0.97 mmol) was hydrogenated to give trans-4-{4-[Methyl-(3-phenyl-propyl)-amino]-cyclohexyl}-phenol (173 mg; 55 %) as white crystals. Mp. 156-158°, MS: m/e=324.4 (M+H⁺).

### Example 40

### trans-4-{4-[Ethyl-(3-phenyl-propyl)-amino]-cyclohexyl}-phenol

### a) trans-N-[4-(4-Benzyloxy-phenyl)-cyclohexyl]-N-(3-phenyl-propyl)-acetamide

A mixture of trans-[4-(4-benzyloxy-phenyl)-cyclohexyl]-(3-phenyl-propyl)-amine (0.4 g, 1.0 mmol), pyridine (4 ml) and acetic anhydride (2 ml) was stirred at 0° for 1 h. Evaporation of the solvents and crystallization of the residue with hexane, gave trans-N-[4-(4-benzyloxy-phenyl)-cyclohexyl]-N-(3-phenyl-propyl)-acetamide (0.26 g, 59%) as white crystals. Mp. 80.0-81.5°, MS: m/e=441 (M⁺).

### b) trans-[4-(4-Benzyloxyl]-phenyl)-cyclohexyl]-ethyl-(3-phenyl-propyl)-amine

To a mixture of LiAlH₄ (74 mg, 1.95 mmol) and THF (10 ml) at 0° under Argon, a solution of trans-N-[4-(4-benzyloxy-phenyl)-cyclohexyl]-N-(3-phenyl-propyl)-acetamide (0.43 g, 0.97 mmol) in THF (10 ml) was added. The suspension was stirred for 2 h at RT and for 1 h at reflux. After cooling to 0°, H₂O (0.4 ml) was carefully added, followed by 15% NaOH (0.4 ml) and H₂O (0.4 ml). The precipitate was removed by filtration and the filtrate dried over Na₂SO₄, and evaporated. The residue was purified by chromatography over SiO₂ (Merck 230-400 mesh) eluting with CH₂Cl₂-MeOH 95:5 to give trans-[4-(4-benzyloxyphenyl)-cyclohexyl]-ethyl-(3-phenyl-propyl)-amine (0.11 g, 26%) as a colorless oil. MS: m/e=328.5 (M+H⁺).

### c) trans-4-{4-[Ethyl-(3-phenyl-propyl)-amino]-cyclohexyl}-phenol

Following the general method of example 22b, 100 mg trans-[4-(4-benzyloxy-phenyl)-cyclohexyl]-ethyl-(3-phenyl-propyl)-amine was hydrogenated to trans-4-{4-[ethyl-(3-phenyl-propyl)-amino]-cyclohexyl}-phenol (16 mg; 20 %) as white crystals. Mp. 163-166°, MS: m/e=338.3 (M+H⁺).

### Example 41

### trans-4-{4- [Methyl-(2-phenoxy-ethyl)-amino]-cyclohexyl} -phenol

### a) trans-[4-(4-Benzyloxy-phenyl)-cyclohexyl]-methyl-(2-phenoxy-ethyl)-amine

Following the general method of example 39a, trans-[4-(4-benzyloxy-phenyl)-cyclohexyl]-(2-phenoxy-ethyl)-amine (300 mg) was N-methylated to give trans-[4-(4-benzyloxyphenyl)-cyclohexyl]-methyl-(2-phenoxy-ethyl)-amine (296 mg; 95 %) as light yellow crystals. Mp. 67-69°, MS: m/e=316.3 (M+H⁺).

### b) trans-4-{4-[Methyl-(2-phenoxy-ethyl)-amino]-cyclohexyl}-phenol

Following the general method of example 22b, trans-[4-(4-benzyloxy-phenyl)-cyclohexyl]-methyl-(2-phenoxy-ethyl)-amine (200 mg) was hydrogenated to give trans-4-{4-[methyl-(2-phenoxy-ethyl)-amino]-cyclohexyl}-phenol (83 mg; 53 %) as white crystals. Mp. 90-92°, MS: m/e=326.3 (M+H⁺).

### Example 42

### cis-4-[4-(2-Phenoxy-ethylamino)-cyclohexyl]-phenol

Following the general method of example 22b, cis-[4-(4-benzyloxy-phenyl)-cyclohexyl]-(2-phenoxy-ethyl)-amine (120 mg) was hydrogenated to give cis-4-[4-(2-phenoxy-ethylamino)-cyclohexyl]-phenol (69 mg; 92 %) as light yellow crystals. Mp. 149-154°, MS: m/e=312.2 (M+H⁺).

### Example 43

### trans-4-[4-(2-Phenoxy-ethylamino)-cyclohexyl-phenol

### a) Cis-[4-(4-Benzyloxy-phenyl)-cyclohexyl]-(2-phenoxy-ethyl)-amine and Trans-[4-(4-Benzyloxy-phenyl)-cyclohexyl-(2-phenoxy-ethyl)-amine

A mixture of 4-(4-benzyloxy-phenyl)-cyclohexanone (8.2 g, 29 mmol), benzyloxyhydroxylamine hydrochloride (4.7 g, 29 mmol) and ethanol was heated for 2 h. at reflux. The solvent was removed and the residue was crystallized to give white crystals (10.2 g, 90%) of the benzyloxy-oxime of 4-(4-benzyloxy-phenyl)-cyclohexanone. The latter was refluxed for 4 days with LiAlH₄ (4 g, 105 mmol) in THF (300 ml). After the addition of H₂O (30 ml), NaOH 15% (30 ml) and H₂O (30 ml), the solids were removed by filtration and the filtrate evaporated. The residue was partioned between H₂O and methylene chloride and the organic layer was dried and evaporated. The residue was purified by chromatography (SiO₂, CH₂Cl₂-MeOH-aq.NH₃ 140:10:1) to give a mixture of cis and trans 4-(4-benzyloxy-phenyl)-cyclohexyl-amine (3.8 g) which was not separated at this stage. A portion of this mixture (1 g, 3.5 mmol) together with 2-phenoxyethyl bromide (0.78 g, 3.55 mmol), K₂CO₃ (1 g) and 2-butanone (20 ml) was refluxed for 3 days. The mixture was evaporated and H₂O was added. Extraction with CH₂Cl₂ gave after drying and evaporation of the solvent a residue which was purified by chromatography (SiO₂, CH₂Cl₂-MeOH 98:2) to give cis-[4-(4-benzyloxy-phenyl)-cyclohexyl]-(2-phenoxy-ethyl)-amine (140 mg, colorless oil, MS: m/e=402.5 (M+H⁺)) and trans-[4-(4-benzyloxy-phenyl)-cyclohexyl]-(2-phenoxy-ethyl)-amine (200 mg, white solid, Mp. 95-98°, MS: m/e=402.5 (M+H⁺)).

### b) trans-4-[4-(2-Phenoxy-ethylamino)-cyclohexyl]-phenol

Following the general method of example 22b, trans- [4-(4-benzyloxy-phenyl)-cyclohexyl]-(2-phenoxy-ethyl)-amine was hydrogenated to give trans-4-[4-(2-phenoxy-ethylamino)-cyclohexyl]-phenol as white crystals. Mp. 160-162°, MS: m/e=312.2 (M+H⁺).

### Example 44

### trans-4-[4-[Methyl-(2-p-tolyloxy-ethyl)-amino]-cyclohexyl]-phenol

### a) trans-[4-(4-Benzyloxy-phenyl)-cyclohexyl]-methyl-(2-p-tolyloxy-ethyl)-amine

A mixture of trans-2-{[4-(4-benzyloxy-phenyl)-cyclohexyl]-methyl-amino}-ethanol (0.3 g, 0.88 mmol), p-cresol (96 mg, 0.88 mmol), triphenylphosphine (232 mg, 0.88 mmol), diethyl-azodicarboxylate (154 mg, 0.88 mmol) and THF was stirred at rt for 3h. The solvent was evaporated and the rsidue was treated with MeOH/H₂O (3:1, 50 ml) and hexane (50 ml). The aq. layer was extracted with hexane (30 ml). The combined hexane extracts were dried and evaporated. The residue was purified by chromatography (SiO₂, CH₂Cl₂-MeOH 95:5) to give trans-[4-(4-benzyloxy-phenyl)-cyclohexyl]-methyl-(2-p-tolyloxy-ethyl)-amine (135 mg, 69 %, white solid, MS: m/e=430.5 (M+H⁺)).

### b) trans-4-[4-[Methyl-(2-p-tolyloxy-ethyl)-amino]-cyclohexyl]-phenol

Following the general method of example 22b, trans-[4-(4-benzyloxy-phenyl)-cyclohexyl]-methyl-(2-p-tolyloxy-ethyl)-amine (200 mg) was hydrogenated to give trans-4-[4-[methyl-(2-p-tolyloxy-ethyl)-amino]-cyclohexyl]-phenol (110 mg; 70 %) as white crystals. MS: m/e=340.3 (M+H⁺).

### Example 45

### cis-4-[4-[Methyl-(2-p-tolyloxy-ethyl)-aminol-cyclohexyl]-phenol

### a) cis-[4-(4-Benqloxy-phenyl)-cyclohexyl]-methyl-(2-p-tolyloxy-ethyl)-amine

Following the general procedure of example 44a, cis-2-{[4-(4-benzyloxy-phenyl)-cyclohexyl]-methyl-amino}-ethanol (100 mg)and p-cresol (32 mg) were converted to cis-[4-(4-benzyloxy-phenyl)-cyclohexyl]-methyl-(2-p-tolyloxy-ethyl)-amine (60 mg; 47 %) light yellow oil, MS: m/e=430.5 (M+H⁺)).

### b) cis-4-[4-[Methyl-(2-p-tolyloxy-ethyl)-amino]-cyclohexy]-phenol

Following the general method of example 22b, cis-[4-(4-benzyloxy-phenyl)-cyclohexyl]-methyl-(2-p-tolyloxy-ethyl)-amine (60 mg) was hydrogenated to give cis-4-[4-[methyl-(2-p-tolyloxy-ethyl)-amino]-cyclohexyl]-phenol (33 mg; 70 %) as a colorless oil. MS: m/e=340.3 (M+H⁺).

### Example 46

### trans-4-(4-{[2-(4-Fluorophenoxy)-ethyl]-methyl-amino}-cyclohexyl)-phenol

### a) trans[4-(4-Benzyloxy-phenyl)-cyclohexyl]-[2-(4-fluoro-phenoxy)-ethyl]-methyl-amine

Following the general procedure of example 44a trans-2-{[4-(4-benzyloxy-phenyl)-cyclohexyl]-methyl-amino}-ethanol (300 mg) and 4-fluoro-phenol (99 mg) were converted to trans[4-(4-benzyloxy-phenyl)-cyclohexyl]-[2-(4-fluoro-phenoxy)-ethyl]-methyl-amine (208 mg; 54 %) light yellow solid, MS: m/e=434.4 (M+H⁺)).

### b) trans-4-(4-{[2-(4-Fluoro-Rhenoxy)-ethyl]-methyl-amino}-cyclohexyl)-phenol

Following the general method of example 22b, trans-[4-(4-benzyloxy-phenyl)-cyclohexyl]-[2-(4-fluoro-phenoxy)-ethyl]-methyl-amine (200 mg) was hydrogenated to give trans-4-(4-{[2-(4-fluoro-phenoxy)-ethyl]-methyl-amino}-cyclohexyl)-phenol as a light yellow solid (110 mg; 69 %. MS: m/e=344.3 (M+H⁺)).

### Example 47

### trans-4-{4-[Methyl-(2-m-tolyloxy-ethyl)-amino]-cyclohexyl}-phenol

### a) trans-[4-(4-Benzyloxy-phenyl)-cyclohexyl]-methyl-(2-m-tolyloxy-ethyl)-amine

Following the general procedure of example 44a, trans-2-{[4-(4-benzyloxy-phenyl)-cyclohexyl]-methyl-amino}-ethanol (300 mg) and m-cresol (108 mg) were converted to trans-[4-(4-benzyloxy-phenyl)-cydohexyl]-methyl-(2-m-tolyloxy-ethyl)-amine (148 mg; 39 % light yellow solid, MS: m/e=430.5 (M+H⁺)).

### b) trans-4-{4-[Methyl-(2-m-tolyloxy-ethyl)-amino]-cydohexyl}-phenol

Following the general method of example 22b, trans-[4-(4-benzyloxy-phenyl)-cyclohexyl]-methyl-(2-m-tolyloxy-ethyl)-amine (140 mg) was hydrogenated to give trans-4-{4-[methyl-(2-m-tolyloxy-ethyl)-amino]-cyclohexyl}-phenol (110 mg; 99 %) as a light yellow solid. MS: m/e=340.3 (M+H⁺).

### Example 48

### trans-4-{4- [methyl-(2-o-tolyloxy-ethyl)-aminol -cyclohexyl} -phenol

### a) cis-2-{[4-4-Benzyloxy-phenyl)-cyclohexyl]-methyl-amino}-ethanol and trans-2-{[4-(4-Benzyloxy-phenyl)-cyclohexyl]-methyl-amino}-ethanol

Following the general method of example 3, cis-2-{ [4-(4-benzyloxy-phenyl)-cyclohexyl]-methyl-amino}-ethanol, (193 mg; 7 % light yellow solid, MS: m/e=340.3 (M+H+)) trans-2-{[4-(4-benzyloxy-phenyl)-cyclohexyl]-methyl-amino}-ethanol (1.3 g; 45 % white crystals, MS: m/e=340.3 (M+H⁺)) were prepared from 4-(4-benzyloxy-phenyl)-cyclohexanone (2.4 g; 8.6 mmol) and 2-methylamino-ethanol (0.64 g; 8.6 mmol).

### b) trans-[4-(4-Benzyloxy-phenyl)-cyclohexyl]-methyl-(2-o-tolyloxy-ethyl)-amine

Following the general procedure of example 44a, trans-2-{[4-(4-benzyloxy-phenyl)-cyclohexyl]-methyl-amino}-ethanol (300 mg) and o-cresol (96 mg) were converted to trans-[4-(4-benzyloxy-phenyl)-cyclohexyl]-methyl-(2-o-tolyloxy-ethyl)-amine (187 mg; 49 % light yellow solid, MS: m/e=430.5 (M+H⁺)).

### c) trans-4-{4-[Methyl-(2-o-tolyloxy-ethyl)-amino]-cyclohexyl}-phenol

Following the general method of example 22b, trans-[4-(4-benzyloxy-phenyl)-cyclohexyl]-methyl-(2-o-tolyloxy-ethyl)-amine (170 mg) was hydrogenated to give trans-4-{4-[methyl-(2-o-tolyloxy-ethyl)-amino]-cyclohexyl}-phenol as a white solid.(120 mg; 89 %; MS: m/e=340.3 (M+H⁺)).

### Example 49

### (RS)-1-[cis-4-(4-Benzyloxy-phenyl)-cyclohexylamino]-3-phenyl-propan-2-ol and (RS)-1-[trans-4-(4-Benzyloxy-phenyl)-cyclohexylamino]-3-phenyl-propan-2-ol

### a) (RS)-1-[cis-4-(4-Benzyloxy-phenyl)-cyclohexylamino]-3-phenyl-propan-2-ol and (RS)-1-[trans-4-(4-Benzyloxy-phenyl)-cyclohexylamino]-3-phenyl-propan-2-ol

A mixture of cis and trans 4-(4-benzyloxy-phenyl)-cyclohexyl-amine (preparation see under 43a, 1.0 g, 3.55 mmol), MeOH (100 ml) and 2,3-epoxypropyl-benzene (480 mg, 3.55 mmol) was refluxed for 3 days. Another portion of and 2,3-epoxypropyl-benzene (480 mg, 3.55 mmol) was added and stirring was continued for 2 days. The solvent was evaporated and the residue was purified by chromatography (SiO₂, AcOEt-hexane 1:1) to give (RS)-1-[cis-4-(4-benzyloxy-phenyl)-cydohexylamino]-3-phenyl-propan-2-ol (430 mg, colorless oil, MS: m/e=416.3 (M+H⁺)) and (RS)-1-[trans-4-(4-benzyloxy-phenyl)-cyclohexylamino]-3-phenyl-propan-2-ol (85 mg, white crystals, Mp. 137.5-138, MS: m/e=416.3 (M+H⁺)).

### b) (RS)-4-[cis-4-(2-Hydroxy-3-phenyl-propylamino)-cyclohexyl-phenol

Following the general method of example 22b, (RS)-1-[cis-4-(4-benzyloxy-phenyl)-cyclohexylamino]-3-phenyl-propan-2-ol (410 mg; 0.99 mmol) was hydrogenated to give (RS)-4-[cis-4-(2-hydroxy-3-phenyl-propylamino)-cyclohexyl]-phenol (240 mg; 75 %) as white crystals. Mp. 52-57°, MS: m/e=326.4 (M+H⁺).

### Example 50

### (RS)-4-[trans-4-(2-Hydroxy-3-phenl-propylamino)-cyclohexyl]-phenol

Following the general method of example 22b, (RS)-1-[trans-4-(4-benzyloxy-phenyl)-cyclohexylamino]-3-phenyl-propan-2-ol (65 mg; 0.16 mmol) was hydrogenated to give (RS)-4-[trans-4-(2-hydroxy-3-phenyl-propylamino)-cyclohexyl]-phenol (39 mg; 77 %) as white crystals. Mp. 56-62°, MS: m/e=326.5 (M+H⁺).

### Example 51

### cis-(4-Phenyl-cyclohexyl)-(3-phenyl-propyl)-amine and trans-(4-phenyl-cyclohexyl)-(3-phenyl-propyl)-amine

Following the general method of example 3, (but using AcOEt-hexane 1:1 for the chromatography), cis-(4-phenyl-cyclohexyl)-(3-phenyl-propyl)-amine, (330 mg; 7 %) light yellow oil, MS: m/e=294.4 (M+H⁺)) and trans-(4-phenyl-cyclohexyl)-(3-phenylpropyl)-amine (660 mg; 20 % white solid, MS: m/e=294.4 (M+H⁺)) were prepared from 4-phenyl-cyclohexanone (2 g; 11.5 mmol) and 3-phenyl-propylamine (1.55 g; 11.5 mmol).

### Example 52

### cis-[4-(4-Nitro-phenyl)-cyclohexyl]-(3-phenyl-propyl)-amine and trans- [4-4-Nitrophenyl)-cyclohexyl]-(3-phenyl-propyl)-amine

Following the general method of example 3, cis-[4-(4-nitro-phenyl)-cyclohexyl]-(3-phenyl-propyl)-amine, (orange oil, MS: m/e=294.4 (M+H⁺)) and trans-[4-(4-nitrophenyl)-cyclohexyl]-(3-phenyl-propyl)-amine (light pink crystals, MS: m/e=294.4 (M+H+)) were prepared from 4-(4-nitro-phenyl)-cyclohexanone and 3-phenyl-propylamine (6.2 g; 45.6 mmol). The trans isomer (9.3 g; 60 %) could be removed from the crude mixture of isomers by crystallization with ether. The mother liquor was then purified by chromatography as described in example 3 to give the pure cis isomer (2.8 g; 18 %).

### Example 53

### trans-4-[4-(3-Phenyl-propylamino)-cyclohexyl]-phenylamine

Following the general method of example 22b, trans-[4-(4-nitro-phenyl)-cyclohexyl]-(3-phenyl-propyl)-amine (200 mg; 0.59 mmol) was hydrogenated to give trans-4-[4-(3-phenyl-propylamino)-cyclohexyl]-phenylamine (160 mg; 88 %) as light orange crystals. Mp. 60-63°, MS: m/e=309.3 (M+H⁺).

### Example 54

### trans-Methyl-[4-(4-nitro-phenyl)-cyclohexyl]-(3-phenyl-propyl)-amine

Following the general method of example 39a, trans-[4-(4-nitro-phenyl)-cyclohexyl]-(3-phenyl-propyl)-amine (3.0 g; 8.9 mmol) was N-methylated to give trans-methyl-[4-(4-nitro-phenyl)-cyclohexyl]-(3-phenyl-propyl)-amine (2.2 g; 70 %) as light yellow crystals. Mp. 55-56°, MS: m/e=353.3 (M+H⁺).

### Example 55

### trans-4-{4-[Methyl-(3-phenyl-propyl)-amino]-cyclohexyl}-phenylamine

Following the general method of example 22b, trans-methyl-[4-(4-nitro-phenyl)-cyclohexyl]-(3-phenyl-propyl)-amine (1.6 g; 4.5 mmol) was hydrogenated to give trans-4-{4-[methyl-(3-phenyl-propyl)-amino]-cyclohexyl}-phenylamine (1.35 g; 92 %) as an orange oil which solidified on standing. MS: m/e=323.4 (M+H⁺).

### Example 56

### trans-N-(4-{4-[Methyl-(3-phenyl-propyl)-amino]-cyclohexyl}-phenyl)-methanesulfonamide

A mixture of trans-4-{4-[methyl-(3-phenyl-propyl)-amino]-cyclohexyl}-phenylamine (300 mg, 093 mmol), pyridine (10 ml) and methanesulfochloride (118 mg, 1.02 mmol) was stirred at 0°. After 2 h additional methanesulfochloride (118 mg, 1.02 mmol) was added, and stirring was continued for 24h. The solvent was evaporated and the residue was purified by chromatography (CH₂Cl₂-MeOH-aq. NH₃ 140:10:1) to give trans-N-(4-{4-[methyl-(3-phenyl-propyl)-amino]-cyclohexyl}-phenyl)-methanesulfonamide (306 mg, 82%) as an orange oil which solidified on standing. MS: m/e=401.6 (M+H⁺).

### Example 57

### (RS)-4-[trans-4-[Methyl-(1-methyl-3-phenyl-propyl)-amino]-cyclohexyl]-phenol

### a) (RS)-[trans-4-(4-Benzyloxy-phenyl)-cyclohexyl]-methyl-(1-methyl-3-phenyl-propyl)-amine

Following the general method of example 39a, (RS)-[trans-4-(4-benzyloxy-phenyl)-cyclohexyl]-(1-methyl-3-phenyl-propyl)-amine (800 mg; 1.9 mmol) was N-methylated to give (RS)-[trans-4-(4-benzyloxy-phenyl)-cyclohexyl]-methyl-(1-methyl-3-phenyl-propyl)-amine (530 mg; 64 %) as a colorless oil which solidified on standing. Mp. 54.5-55.5°, MS: m/e=428.5 (M+H⁺).

### b) (RS)-4-[trans-4-[Methyl-(1-methlrl-3-phenyl-propyl)-amino]-cyclohexyl]-phenol

Following the general method of example 22b, (RS)-[trans-4-(4-benzyloxy-phenyl)-cyclohexyl]-methyl-(1-methyl-3-phenyl-propyl)-amine (410 mg; 0.96 mmol) was hydrogenated to give (RS)-4-[trans-4-[methyl-(1-methyl-3-phenyl-propyl)-amino]-cyclohexyl]-phenol (165 mg; 51 %) as white crystals. MS: m/e=338.3 (M+H⁺).

### Example 58

### 4-[trans-4-[(RS)-1-Methyl-3-phenyl-propylamino)-cydohexyl]-phenol

Following the general method of example 22b, (RS)-[trans-4-(4-benzyloxy-phenyl)-cyclohexyl]-(1-methyl-3-phenyl-propyl)-amine (300 mg; 0.72 mmol) was hydrogenated to give 4-[trans-4-[(RS)-1-methyl-3-phenyl-propylamino)-cyclohexyl]-phenol (168 mg; 72 %) as white crystals. Mp. 155-160°, MS: m/e=324.4 (M+H⁺).

### Example 59

### 4-[cis-4-[(RS)-1-Methyl-3-phenyl-propylamino)-cyclohexyl]-phenol

### a) (RS)-[cis-4-(4-Benzyloxy-phenyl)-cyclohexyl]-(1-methyl-3-phenyl-3-propyl)-amine and (RS)-[trans-4-(4-Benzyloxy-phenyl)-cyclohexyl]-(1-methyl-3-phenyl-propyl)-amine

Following the general method of example 3, (RS)-[cis-4-(4-benzyloxy-phenyl)-cyclohexyl]-(1-methyl-3-phenyl-propyl)-amine (1.90 g; 43 % light brown oil, MS: m/e=414.4 (M+H⁺)) and (RS)-[trans-4-(4-benzyloxy-phenyl)-cyclohexyl]-(1-methyl-3-phenyl-propyl)-amine (1.26 g; 29 % light yellow solid, Mp. 70-72°, MS: m/e=414.4 (M+H⁺)) were prepared from 2-(4-methoxyphenyl)-cyclohexanone (3.0 g; 10.7 mmol) and 3-amino-1-phenylbutane (1.6 g; 10.7 mmol).

### b) 4-[cis-4-[(RS)-1-Methyl-3-phenl-propylamino)-cyclohexyl]-phenol

Following the general method of example 22b, (RS)-[cis-4-(4-benzyloxy-phenyl)-cyclohexyl]-(1-methyl-3-phenyl-propyl)-amine (1.6 g; 3.9 mmol) was hydrogenated to give 4-[cis-4-[(RS)-1-methyl-3-phenyl-propylamino)-cyclohexyl]-phenol (369 mg; 30 %) as white crystals. Mp. 44-49°, MS: m/e=324.4 (M+H⁺).

### Example 60

### trans-4-[4-(3-p-Tolyl-propylamino)-cyclohexyl]-phenol

### a) cis-[4-(4-Benzyloxy-phenyl)-cyclohexyl]-(3-p-tolyl-propyl)-amine and trans-[4-(4-Benzyloxy-phenyl)-cyclohexyl]-(3-p-tolyl-propyl)-amine

A mixture of cis and trans 4-(4-benzyloxy-phenyl)-cyclohexyl-amine (preparation see under example 43a, 1.25 g, 4.44 mmol), K₂CO₃ (1.23 g, 8.88 mmol), 1-(3-Bromo-propyl)-4-methyl-benzene (1.18 g, 4.44 mmol) and 2-butanone was stirred at 80° for 48 h. Water was then added and the products were extracted with EtOAc. The organic layer was dried (Na₂SO₄), evaporated and the residue was purified by chromatography (SiO₂, CH₂Cl₂₋MeOH-aq. NH₃ 140:10:1) to give cis-[4-(4-benzyloxy-phenyl)-cyclohexyl]-(3-p-tolyl-propyl)-amine (180 mg; 10 %) as a light yellow solid (MS: m/e=414.4 (M+H⁺))and trans-[4-(4-benzyloxy-phenyl)-cyclohexyl]-(3-p-tolyl-propyl)-amine (38 mg; 2 %) as a light yellow solid (MS: m/e=414.4 (M+H⁺)).

### b) trans-4-[4-(3-p-Tolyl-propylamino)-cyclohexyl]-phenol

Following the general method of example 22b, trans-[4-(4-benzyloxy-phenyl)-cyclohexyl]-(3-p-tolyl-propyl)-amine (35 mg) was hydrogenated to give trans-4-[4-(3-p-Tolyl-propylamino)-cyclohexyl]-phenol (20 mg; 73 %) as a white solid. MS: m/e=324.4 (M+H⁺).

### Example 61

### trans-4-(4-{[3-(4-Fluoro-phenyl)-propyl]-methyl-amino}-cyclohexyl)-phenol

### a) trans-[4-(4-Benzyloxy-phenyl)-cyclohexyl]-[3-(4-fluoro-phenyl)-propyl]-methylamine

Following the general method of example 39a, trans-[4-(4-benzyloxy-phenyl)-cyclohexyl]-[3-(4-fluoro-phenyl)-propyl]-amine (700 mg) was N-methylated to give trans-[4-(4-benzyloxy-phenyl)-cyclohexyl]-[3-(4-fluoro-phenyl)-propyl]-methyl-amine as a light yellow solid (650 mg; 90 %), MS: m/e=432.4 (M+H⁺)).

### b) trans-4-(4-{[3-(4-Fluoro-phenyl)-propyl}-methyl-amino}-cyclohexyl)-phenol

Following the general method of example 22b, trans-[4-(4-benzyloxy-phenyl)-cyclohexyl]-[3-(4-fluoro-phenyl)-propyl]-methyl-amine(600 mg) was hydrogenated to give trans-4-(4-{[3-(4-nuoro-phenyl)-propyl]-methyl-amino}-cydohexyl)-phenol (200 mg; 42 %) as a white solid. MS: m/e=342.3 (M+H⁺).

### Example 62

### trans-4-[4- [3-(4-Fluoro-phenyl)-propylamino]-cyclohexyl]-phenol

### a) trans-[4-(4-Benzyloxy-phenyl)-cyclohexyl]-[3-(-4-fluoro-phenyl)-propyl]-amine

Following the general method of example 3 trans-[4-(4-benzyloxy-phenyl)-cyclohexyl]-[3-(4-fluoro-phenyl)-propyl]-amine (135 mg; 13 % white solid, MS: m/e=418.4 (M+H⁺)) was prepared from 2-(4-methoxyphenyl)-cyclohexanone (0.7 g) and 3-(4-fluorophenyl)propylamine (0.4 g). In this case the cis isomer was not isolated.

### b) trans-4-[4-[3-(4-Fluoro phenyl)-propylaminol-cyclohexyl]-uhenol

Following the general method of example 22b, trans-[4-(4-benzyloxy-phenyl)-cyclohexyl]-[3-(4-fluoro-phenyl)-propyl]-amine (130 mg) was hydrogenated to give trans-4-[4-[3-(4-fluoro-phenyl)-propylamino]-cyclohexyl]-phenol (100 mg; 98 %) as a white solid. MS: m/e=328.3 (M+H⁺).

### Example 63

### (RS)-4-[trans-4-[(3-Hydroxy-3-phenyl-propyl)-methyl-amino]-cyclohexyl]-phenol

### a) (RS)-3-[trans-4-(4-Benzyloxy-phenyl)-cyclohexyl]-methy]-amino]-1-phenyl-propan-1-ol

Following the general method of example 39a, (RS)-3-[trans-4-(4-benzyloxy-phenyl)-cyclohexylamino]-1-phenyl-propan-1-ol (300 mg) was N-methylated to give (RS)-3-[[trans-4-(4-benzyloxy-phenyl)-cyclohexyl]-methyl-amino]-1-phenyl-propan-1-ol (270 mg; 87 %) as a white solid. MS: m/e=430.5 (M+H⁺).

### b) (RS)-4-[trans-4-[(3-Hydroxy-3-phenyl-propyl)-methyl-aminol-cyclohexyl]-phenol

Following the general method of example 22b, (RS)-3- [[trans-4-(4-benzyloxy-phenyl)-cyclohexyl]-methyl-amino]-1-phenyl-propan-1-ol (200 mg) was hydrogenated to give (RS)-4-[trans-4-[(3-hydroxy-3-phenyl-propyl)-methyl-amino]-cydohexyl]-phenol as a white solid (150 mg; 95 %). MS: m/e=340.3 (M+H⁺).

### Example 64

### (RS)-4-[trans-4-(3-Hydroxy-3-phenyl-propylamino)-cyclohexyl]-phenol

### a) (RS)-3-[trans-4-(4-Benzyloxy-phenyl)-cyclohexylamino]-1-phenyl-propan-1-ol

Following the general method of example 3 (RS)-3-[trans-4-(4-benzyloxy-phenyl)-cyclohexylamino]-1-phenyl-propan-1-ol (840 mg; 10 %) white crystals, MS: m/e=416.3 (M+H⁺)) was prepared from 2-(4-methoxyphenyl)-cyclohexanone (5.56 g) and 3-hydroxy-3-phenyl-propylamine (3.0 g) (for the preparation of this amine: see T. M. Koenig, D. Mitchell, Tetrahedron Letters, 1994, 1339-1342). In this case, the cis isomer was not isolated.

### b) (RS)-4-trans-4-(3-Hydroxy-3-phenyl-propylamino)-cydohexyl]-phenol

Following the general method of example 22b, (RS)-3-[trans-4-(4-benzyloxy-phenyl)-ryclohexylamino]-1-phenyl-propan-1-ol (200 g) was hydrogenated to give (RS)-4-[trans-4-(3-hydroxy-3-phenyl-propylamino)-cyclohexyl]-phenol (120 mg; 77 %) as a white solid. MS: m/e=326.3 (M+H⁺).

### Example 65

### (RS)-4-[trans-4-[(1-Hydroxymethyl-3-phenyl-propyl)-methyl-amino]-cyclohexyl-phenol

### a) (RS)-2-[[trans-4-(4-Benzyloxy-phenyl)-cycloxyl]-methyl-amino]-4-phenyl-butan-1-ol

Following the general method of example 39a, (RS)-2-[trans-4-(4-benzyloxy-phenyl)-cyclohexylamino]-4-phenyl-butan-1-ol (500 mg, 1.116 mmol) was N-methylated to give (RS)-2-[[trans-4-(4-benzyloxy-phenyl)-cyclohexyl]-methyl-amino]-4-phenyl-butan-1-ol as a colorless oil (357 mg, 69%, MS: m/e=444.4 (M+H⁺)).

### b) (RS)-4-[trans-4-[(1-Hydroxymethyl-3-phenyl-propyl)-methyl-amino]-cyclohexyl]-phenol

Following the general method of example 22b, (RS)-2-[[trans-4-(4-benzyloxy-phenyl)-ryclohexyl]-methyl-amino]-4-phenyl-butan-1-ol (250 mg, 0.56 mmol) was hydrogenated to give (RS)-4-[trans-4-[(1-hydroxymethyl-3-phenyl-propyl)-methyl-amino]-cyclohexyl]-phenol as a white solid (190 mg, 95%, MS: m/e=354.4 (M+H⁺).

### Example 66

### (RS)-4-[trans-4-(1-Hydroxymethyl-3-phenyl-propylamino)-cyclohexyl]-phenol

### a) (RS)-2-Amino-4-phenyl-butan-1-ol

Following the general procedure of example 40b, DL-homophenylalanine (5.0 g, 27.9 mmol) was reduced with LiAlH₄ to give (RS)-2-amino-4-phenyl-butan-1-ol (3.6 g, 78%, MS: m/e=166 (M+H⁺)) as a light yellow solid.

### b) (RS)-2-[cis-4-(4-Benzyloxy-phenyl)-cyclohexylamino]-4-phenyl-butan-1-ol and (RS)-2-[trans-4-(4-Benzyloxy-phenyl)-cyclohexylamino]-4-phenyl-butan-1-ol

Following the general method of example 3 (RS)-2-[cis-4-(4-benzyloxy-phenyl)-cyclohexylamino]-4-phenyl-butan-1-ol (white crystals, 600 mg, 6.6%, MS: m/e=430.5 (M+H⁺)) and (RS)-2-[trans-4-(4-benzyloxy-phenyl)-cyclohexylamino]-4-phenyl-butan-1-ol (4.5 g, 50%, white crystals, MS: m/e=430.5 (M+H⁺)) were prepared from 2-(4-methoxyphenyl)-cyclohexanone (5.9 g, 21 mmol) and (RS)-2-amino-4-phenyl-butan-1-ol (3.48 g, 21 mmol).

### c) (RS)-4-[trans-4-(1-Hydroxymethyl-3-phenyl-propylamino)-cydohexyl]-phenol

Following the general method of example 22b, (RS)-2-[trans-4-(4-benzyloxy-phenyl)-cyclohexylamino]-4-phenyl-butan-1-ol (250 mg, 0.58 mmol) was hydrogenated to give (RS)-4-[trans-4-(1-hydroxymethyl-3-phenyl-propylamino)-cyclohexyl]-phenol as a white solid (160 mg, 81%, MS: m/e=340.3 (M+H⁺)).

### Example 67

### (RS)-4-[trans-4-[(4-Hydroxy-3-phenyl-butyl)-methyl-amino]-cyclohexyl]-phenol

### a) (RS)-4-[[trans-4-(4-Benzyloxy-phenyl)-cyclohexyl-methyl-amino]-2-phenyl-butan-1-ol

Following the general method of example 39a, (RS)-4-[trans-4-(4-benzyloxy-phenyl)-cydohexylamino]-2-phenyl-butan-1-ol (500 mg, 1.16 mmol) was N-methylated to give (RS)-4- [[trans-4-(4-benzyloxy-phenyl)-cyclohexyl] -methyl-amino]-2-phenyl-butan-1-ol as a colorless oil (285 mg, 55%, MS: m/e=444.4 (M+H⁺)).

### b) (RS)-4-[trans-4-[(4-Hydroxy-3-phenyl-butyl)-methyl-amino]-cyclohexyl]-phenol

Following the general method of example 22b, (RS)-4-[[trans-4-(4-benzyloxy-phenyl)-cyclohexyl]-methyl-amino]-2-phenyl-butan-1-ol (240 mg, 0.54 mmol) was hydrogenated to give (RS)-4- [trans-4- [(4-hydroxy-3-phenyl-butyl)-methyl-amino]-cyclohexyl]-phenol as white crystals (79 mg, 41%, MS: m/e=354.4 (M+H⁺)).

### Example 68

### (RS)-4-[trans-4-(4-Hydroxy-3-phenyl-butylamino)-cyclohexyl]-phenol

### a) (RS)-4-[cis-4-(4-Benzyloxy-phenyl)-cyclohexylamino]-2-phenyl-butan-1-ol and (RS)-4-[trans-4-(4-Benzyloxy-phenyl)-cyclohexylamino]-2-phenyl-butan-1-ol

Following the general method of example 3 (RS)-4-[cis-4-(4-benzyloxy-phenyl)-cyclohexylamino]-2-phenyl-butan-1-ol (white solid, 910 mg, 12%, MS: m/e=430.5 (M+H⁺)) and (RS)-4-[trans-4-(4-benzyloxy-phenyl)-cyclohexylamino]-2-phenyl-butan-1-ol (940 mg, 13%, white crystals, Mp. 140-142°, MS: m/e=430.5 (M+H⁺)) were prepared from 2-(4-methoxyphenyl)-cyclohexanone (4.9 g, 17.55 mmol) and (RS)- 4-Amino-2-phenyl-butan-1-ol (2.9 g, 17.55 mmol).

### b) (RS)-4-[trans-4-(4-Hydroxy-3-phenyl-butylamino)-cyclohexyl]-phenol

Following the general method of example 22b, (RS)-4-[trans-4-(4-benzyloxy-phenyl)-cyclohexylamino]-2-phenyl-butan-1-ol (300 mg, 0.67 mmol) was hydrogenated to give (RS)-4-[trans-4-(4-hydroxy-3-phenyl-butylamino)-ryclohexyl]-phenol as a white crystals (210 mg, 89%, MS: m/e=340.3 (M+H⁺)).

### Example 69

### (RS)-4-[trans-4-[(2-Hydroxymethyl-3-phenyl-propyl)-methyl-amino]-cyclohexyl]-phenol

### a) (RS)-2-Benzyl-3-[[trans-4-(4-benzyloxy-phenyl)-cyclohexyl]-methyl-amino]-propan-1-ol

Following the general method of example 39a, (RS)-2-benzyl-3-[trans-4-(4-benzyloxyphenyl)-cyclohexylamino]-propan-1-ol (300 mg, 0.7 mmol) was N-methylated to give (RS)-2-benzyl-3-[[trans-4-(4-benzyloxy-phenyl)-cyclohexyl]-methyl-amino]-propan-1-ol as a colorless oil (300 mg, 97%, MS: m/e=444.4 (M+H⁺)).

### b) (RS)-4-[trans-4-[(2-Hydroxymethyl-3-phenyl-propyl)-methyl-amino]-cyclohexyl]-phenol

Following the general method of example 22b, (RS)-2-benzyl-3-[[trans-4-(4-benzyloxy-phenyl)-cyclohexyl]-methyl-amino]-propan-1-ol (250 mg, 0.56 mmol) was hydrogenated to give (RS)-4-[trans-4-[(2-hydroxymethyl-3-phenyl-propyl)-methyl-amino]-cyclohexyl]-phenol as white crystals (190 mg, 95%, MS: m/e=354.3 (M+H⁺)).

### Example 70

### (RS)-4-[trans-4-(2-Hydroxymethyl-3-phenyl-propylamino)-cyclohexyl]-phenol

### a) (RS)-2-Benzyl-3-[cis-4-(4-benzyloxy-phenyl)-cydohexylamino]-propan-1-ol and (RS)-2-Benzyl-3-[trans-4-(4-benzyloxy-phenyl)-cyclohexylamino]-propan-1-ol

Following the general method of example 3 (RS)-2-benzyl-3-[cis-4-(4-benzyloxy-phenyl)-cyclohexylamino]-propan-1-ol(white solid, 870 mg, 12%, MS: m/e=430.5 (M+H⁺)) and (RS)-2-benzyl-3-[trans-4-(4-benzyloxy-phenyl)-cyclohexylamino]-propan-1-ol (2.83 g, 38%, white crystals, MS: m/e=430.5 (M+H⁺)) were prepared from 2-(4-methoxyphenyl)-cyclohexanone (4.9 g, 17.5 mmol) and (RS)-3-amino-2-benzyl-propan-1-ol (2.9 g, 17.5 mmol).

### b) (RS)-4-[trans-4-(2-Hydroxymethyl-3-phenyl-propylamino)-cyclohexyl]-phenol

Following the general method of example 22b, (RS)-2-benzyl-3-[trans-4-(4-benzyloxyphenyl)-cyclohexylamino]-propan-1-ol (250 mg, 0.58 mmol) was hydrogenated to give (RS)-4- [trans-4-(2-hydroxymethyl-3-phenyl-propylamino)-cydohexyl]-phenol as a white solid (184 mg, 93%, MS: m/e=340.3 (M+H⁺)).

### Example 71

### cis-4-[1-Hydroxy-4-(3-phenyl-propylamino)-cydohexyl]-phenol

### a) 4-(4-Benzyloxy-phenyl)-4-hydroxy-cyclohexanone

To a solution of the Grignard reagent prepared from Magnesium turnings (2.33 g, 96 mmol) and 4-benzyloxybromobenzene (25 g, 95 mmol) in THF (150 ml), 1,4-Cyclohexanedione monoethylene ketal (10 g, 64 mmol) in THF (150 ml) was added. After completeion of the reaction, the solution was added to 200 ml of aq. NH₄Cl and extracted with CH₂Cl₂. Drying and evaporation of the organic layer gave a residue which was crystallised from AcOEt-hexane to give 8-(4-benzyloxy-phenyl)-1,4-dioxa-spiro[4.5]decan-8-ol (17.5 g, 54%, MS: m/e=340 (M⁺)). Hydrolysis (72 h at r.t.) of this intermadiate (9 g, 26.4 mmol) with H₂O (80 ml) and trifluoroacetic acid (18.2 ml) gave after extraction with CH₂Cl₂ 4-(4-benzyloxy-phenyl)-4-hydroxy-cyclohexanone (6.39 g, 81.5%, MS: m/e=296 (M⁺)).

### b) cis-1-(4-Benzyloxy-phenyl)-4-(3-phenyl-propylamino)-cyclohexanol

Following the general method of example 3 cis-1-(4-benzyloxy-phenyl)-4-(3-phenyl-propylamino)-cyclohexanol (light yellow solid, 250 mg, 18%, MS: m/e=416.3 (M+H⁺)) was prepared from 4-(4-benzyloxy-phenyl)-4-hydroxy-cyclohexanone (1 g, 3.37 mmol) and 3-phenylpropylamine (0.456 g, 3.37 mmol).

### c) cis-4-[1-Hydroxy-4-(3-phenyl-propylamino)-cyclohexyl]-phenol

Following the general method of example 22b, cis-1-(4-benzyloxy-phenyl)-4-(3-phenyl-propylamino)-cyclohexanol (200 mg, 0.48 mmol) was hydrogenated to cis-4-[1-hydroxy-4-(3-phenyl-propylamino)-cyclohexyl]-phenol as white crystals (from acetonitrile, 85 mg, 55%, MS: m/e=326.4 (M+H⁺)).

### Example 72

### trans-4- 1-Hydroxy-4-(3-phenyl-propylamino)-cyclohexyl]-phenol

### a) (1,4-Dioxa-spiro[4.5]dec-8-yl)-(3-phenyl-propyl)-amine hydrochloride (1:1)

A mixture of cyclohexanedione monoethylene ketal (4 g, 25.6 mmol), 3-phenylpropylamin (3.46 g, 25.6 mmol), pTosOH (catalytic amount) and toluene (100 ml) was refluxed overnight in a Dean-Stark apparatus. The solvent was then evaporated and THF (100 ml), MeOH (10 ml) and NaBH₃CN (1.89 g) were added. The pH was adjusted to 3-4 by HCl/MeOH addition. After stirring for 4 h, the mixture was treated with sat. NaHCO₃ solution (150 ml) and extracted with CH₂Cl₂. The organic layer was evaporated and the residue was purified by chromatography (SiO₂, CH₂Cl₂-MeOH-aq. NH₃ 140:10:1) to give a yellow oil (4.01 g). This was dissolved in MeOH (10 ml) and HCl/MeOH was added dropwise. Ether was added (100 ml) and the white precipitate was collected to afford (1,4-Dioxa-spiro[4.5]dec-8-yl)-(3-phenyl-propyl)-amine hydrochloride (1:1) (3.8 g, 48%, MS: m/e=276.3 (M+H⁺)).

### b) 4-(3-Phenyl-propylamino)-cyclohexanone

A mixture of (1,4-dioxa-spiro[4.5]dec-8-yl)-(3-phenyl-propyl)-amine hydrochloride (1:1) (3.35 g, 10.7 mmol), H₂O (25 ml) and trifluoroacetic acid (2.47 ml, 32.2 mmol) was left at r.t for 48 h. NaHCO₃ (10%, 100 ml) was added and the solution was extracted with CH₂Cl₂. Drying of the organic layer (Na₂SO₄) and evaporation left 4-(3-phenyl-propylamino)-cyclohexanone as a slightly coloured oil (2.4 g, 97%, MS: m/e=232.2 (M+H⁺)).

### c) (4-Oxo-cyclohexyl)-(3-phenyl-propyl)-carbamic acid benzyl ester

A mixture of 4-(3-phenyl-propylamino)-cyclohexanone (1.9 g, 8.21 mmol), K₂CO₃ (1.15 g, 8.3 mmol), benzyl chloroformate (1.42 g, 8.3 mmol) and CH₂Cl₂ (20 ml) was stirred at r.t for 1.5 h. H₂O (50 ml) was added, and the mixture was extracted with CH₂Cl₂. The organic layer was dried (Na₂SO₄), evaporated and the residue was purified by chromatography (SiO₂, CH₂Cl₂-MeOH 95:5) to give (4-Oxo-cyclohexyl)-(3-phenyl-propyl)-carbamic acid benzyl ester as a yellow oil (2.7 g, 90%, MS: m/e=365(M⁺)).

### d) trans-4-[1-Hydroxy-4-(3-phenyl-propylamino)-cyclohexyl]-phenol

To the Grignard reagent prepared from Magnesium turnings (0.3 g, 12.3 mmol) and 4-benzyloxybromobenzene (2.7 g, 10.26 mmol) in THF (100ml) was added (4-Oxo-cyclohexyl)-(3-phenyl-propyl)-carbamic acid benzyl ester (2.5 g, 6.84 mmol) in THF (50 ml). After refluxing for 2 h, cooling to 0°, addition of aq. NH₄Cl (100 ml), extraction with CH₂Cl₂, drying (Na₂SO₄) and evaporation of the organic layer, and purification of the residue by chromatography (SiO₂, CH₂Cl₂-MeOH 98:2) gave the benzylated intermediate (565 mg, 15%). Following the general method of example 22b this intermediate (200 mg, 0.364 mmol) was hydrogenated to give trans-4-[1-hydroxy-4-(3-phenyl-propylamino)-cyclohexyl]-phenol as light yellow crystals (53 mg, 45%, MS: m/e=326.4 (M+H⁺)).

### Example 73

### (1RS,3RS,4RS)-4-[3-Methyl-4-[methyl(3-phenyl-propyl)-aminol]-cyclohexyl]-phenol

### a) (1RS,2RS,4RS)-[4-(4-Benzyloxy-phenyl)-2-methyl-cyclohexyl]-methyl-(3-phenylpropyl)-amine

Following the general method of example 39a, (1RS,2RS,4RS)-[4-(4-benzyloxy-phenyl)-2-methyl-cyclohexyl]-(3-phenyl-propyl)-amine (160 mg, 0.39 mmol) was N-methylated to give (1RS,2RS,4RS)-[4-(4-benzyloxy-phenyl)-2-methyl-cyclohexyl]-methyl-(3-phenylpropyl)-amine as a colorless oil (116 mg, 70 %, MS: m/e=428.6 (M+H⁺)).

### b) (1RS,3RS,4RS)-4-[3-Methyl-4-[methyl-(3-phenyl-propyl)-amino]-cyclohexyl]-phenol

Following the general method of example 22b, (1RS,2RS,4RS)-[4-(4-benzyloxy-phenyl)-2-methyl-cyclohexyl]-methyl-(3-phenyl-propyl)-amine (100 mg, 0.23 mmol) was hydrogenated to give (1RS,3RS,4RS)-4-[3-methyl-4-[methyl-(3-phenyl-propyl)-amino]-cyclohexyl]-phenol as a light-yellow oil (42 mg, 53 %, MS: m/e=338.3 (M+H⁺)).

### Example 74

### (1RS,3RS,4RS)-4-[3-Methyl-4-(3-phenyl-propylamino)-cyclohexyl]-phenol

### a) (2RS,4RS)-4-(4-Benzyloxy-phenyl)-2-methyl-cyclohexanone

To a solution of LDA (23.6 mmol) in THF (80 ml) at -75°, a solution of 4-(4-benzyloxyphenyl)-cyclohexanone (6 g, 21.4 mmol) in THF (50 ml) was added over 20 min. After 1h at -75°, iodomethane (3.2 g, 22.5 mmol) was added and stirring was continued at RT overnight. The solution was then acidified and partitioned between H₂O and AcOEt. Extraction with AcOEt, drying of the organic layers, evaporation and purification of the residue by chromatography (SiO₂, AcOEt-hexane 1:6) gave a first fraction (3.15 g) of monomethyl derivatives and a second fraction (1.02 g) of dimethyl derivatives. From the the first fraction the title compound (2RS,4RS)-4-(4-benzyloxy-phenyl)-2-methylcyclohexanone (1.941 g, white crystals, 28%, MS: m/e=294 (M⁺)) was separated by crystallisation from ether.

### b) (1RS,2RS,4RS)-[4-(4-Benzyloxy-phenyl)-2-methyl-cyclohexyl]-(3-phenyl-propyl)-amine

Following the general method of example 3 (1RS,2RS,4RS)-[4-(4-benzyloxy-phenyl)-2-methyl-cyclohexyl]-(3-phenyl-propyl)-amine (white solid, 301 mg, 21.4%, MS: m/e=414.4 (M+H⁺)) was prepared from (2RS,4RS)-4-(4-benzyloxy-phenyl)-2-methyl-cyclohexanone (1.0 g, 3.4 mmol) and 3-phenylpropylamine (0.46 g, 3.4 mmol).

### c) (1RS,3RS,4RS)-4-[3-Methyl-4-(3-phenyl-propylamino)-cyclohexyl]-phenol

Following the general method of example 22b, (1RS,2RS,4RS)-[4-(4-benzyloxy-phenyl)-2-methyl-cyclohexyl]-(3-phenyl-propyl)-amine (110 mg, 0.27 mmol) was hydrogenated to give (1RS,3RS,4RS)-4-[3-methyl-4-(3-phenyl-propylamino)-cyclohexyl]-phenol as light-yellow crystals (76 mg, 88%, MS: m/e=324.4 (M+H⁺)).

### Example 75

### trans-4-[5-(3-Phenyl-propylamino)-[1,3]dioxan-2-yl]-phenol 1:1 but-2-enedioic acid

Following the general procedure of example 40b, trans-N-[2-(4-hydroxy-phenyl)-[l,3Jdioxan-5-yl]-3-phenyl-propionamide (2.5 g; 7.6 mmol) was reduced to trans-4-[5-(3-phenyl-propylamino)-[l,3]dioxan-2-yl]-phenol (470 mg; 20 %). Treatment with fumaric acid (150 mg) in ether gave the title compound (160 mg; 78 %, MS: m/e=314.3 (M+H⁺)).

### Example 76

### trans-N-[2-(4-Hydroxy-phenyl)-[1,3]dioxan-5-yl]-3-phenyl-propionamide and cis-N-[2-(4-Hydroxy-phenyl)-[1,3]dioxan-5-yl]-3-phenyl-propionamide

### a) N-(2-Hydroxy-1-hydroxymethyl-ethyl)-3-phenyl-propionamide

The procedure of example 79c was followed, using 3-phenylpropionic acid and serinol. At the end of the reaction, the DMF was evaporated and CH₂Cl₂ was added to the residue. The white crystalline material was collected and gave pure N-(2-hydroxy-1-hydroxymethyl-ethyl)-3-phenyl-propionamide as a white solid (56%, MS: m/e=224.2 (M+H⁺)).

### b) trans-N-[2-(4-Hydroxy-phenyl)-[1,3]dioxan-5-yl]-3-phenyl-propionamide and cis-N-[2-(4-Hydroxy-phenyl)-[1,3]dioxan-5-yl]-3-phenyl-propionamide

A mixture of N-(2-hydroxy-1-hydroxymethyl-ethyl)-3-phenyl-propionamide (1.7 g, 7.6 mmol), toluene (70 ml), 4-hydroxybenzaldehyde (1.856 g,15.2 mmol) and a catalytic amount of p-toluenesulfonic acid was refluxed for 3 h. The mixture was evaporated and CH₂Cl₂ was added to the residue. The precipitate was filtered off and recrystallised from hot AcOEt (50 ml). The light brown crystals formed collected yielding trans-N-[2-(4-hydroxy-phenyl)-[1,3]dioxan-5-yl]-3-phenyl-propionamide (1.41 g, MS: m/e=328.2 (M+H⁺)). The filtrate was concentrated and left at 4° overnight. The newly formed crystals are collected yielding cis-N-[2-(4-hydroxy-phenyl)-[1,3]dioxan-5-yl]-3-phenyl-propionamide (0.401 g, m/e=326.4 (M-H⁺)).

### Example A

| Tablet Formulation (Wet Granulation) | | | | | |
|---|---|---|---|---|---|
| Item | Ingredients | mg/tablet | | | |
| | | 5 mg | 25 mg | 100mg | 500mg |
| 1. | Compound of formula 1 | 5 | 25 | 100 | 500 |
| 2. | Lactose Anhydrous DTG | 125 | 105 | 30 | 150 |
| 3. | Sta-Rx 1500 | 6 | 6 | 6 | 30 |
| 4. | Microcrystalline Cellulose | 30 | 30 | 30 | 150 |
| 5. | Magnesium Stearate | 1 | 1 | 1 | 1 |
| | Total | 167 | 167 | 167 | 835 |

### Manufacturing Procedure

1. Mix items 1, 2, 3 and 4 and granulate with purified water.
2. Dry the granulation at 50°C.
3. Pass the granulation through suitable milling equipment.
4. Add item 5 and mix for three minutes; compress on a suitable press.

### Example B

| Capsule Formulation | | | | | | |
|---|---|---|---|---|---|---|
| Item | Ingredients | | | | | |
| | 5 mg | | 25 mg | | 100mg | 500mg |
| 1. | Compound of formula 1 | | 5 | 25 | 100 | 500 |
| 2. | Hydrous Lactose | | 159 | 123 | 148 | --- |
| 3. | Corn Starch | | 25 | 35 | 40 | 70 |
| 4. | Talc | | 10 | 15 | 10 | 25 |
| 5. | Magnesium Stearate | | 1 | 2 | 2 | 5 |
| | Total | 200 | | 200 | 300 | 600 |

### Manufacturing Procedure

1. Mix items 1, 2, and 3 in a suitable mixer for 30 minutes.
2. Add items 4 and 5 and mix for 3 minutes.
3. Fill into a suitable capsule.
4. Add item 5 and mix for three minutes; compress on a suitable press.

## Claims

1. The use of a compound of the general formula 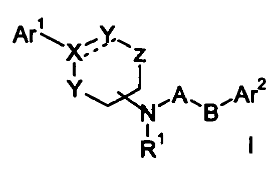 wherein
Ar¹ is phenyl, naphthyl or tetrahydronaphthyl, optionally substituted by hydroxy, C₁-C₄ alkoxy, nitro, amino or methanesulfonamide;
Ar² is phenyl, naphthyl or tetrahydronaphthyl, optionally substituted by C₁-C₄ alkyl or halogen;
X is C, CH or C(OH);
Y is -CH₂-, CH or O
Z -CH₂-, -CH(CH₃)- or -C(CH₃)₂-;
R¹ is hydrogen, C₁-C₄ alkyl or acetyl;
A is C=O or -(CHR²)ₙ-, wherein R² is hydrogen, C₁-C₄ alkyl or hydroxy- C₁-C₄ alkyl;
B is -(CH₂)ₙ-, O, -CH(OH)(CH₂)ₙ-, -CH(CH₂OH)(CH₂)ₙ-, -(CH₂)ₙ CH(OH)- or -CH(CH₂OH)-;
--- may be a bond and
n is 0-4
and pharmaceutically acceptable acid addition salts thereof for the manufacture of medicaments for the treatment of neurodegeneration, caused by stroke, brain trauma, Alzheimer's disease, Parkinson's disease, Huntington's disease, ALS (amyotrophic lateral sclerosis), and neurodegeneration associated with bacterial or viral infections.

2. The use of a compound of the formula Ia in accordance with claim 1 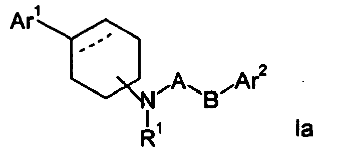 wherein Ar¹, Ar², A, B, R¹ and the dotted line have the significances given in claim 1.

3. The use of a compound according to claim 2, which is
trans-4-[4-(3-phenyl-propylamino)-cyclohexyl] -phenol,
trans-4-[4-[methyl-(3-phenyl-propyl)-amino]-cydohexyl] -phenol,
trans-4-[4- [ethyl-(3-phenyl-propyl)-amino]-cyclohexyl]-phenol,
trans-4-[4-(4-phenyl-butylamino)-cyclohexyl]-phenol,
trans-4-[4- [3-(4-fluoro-phenyl)-propylamino]-cydohexyl] phenol,
trans-4-(4-[[3-(4- fluoro-phenyl)-propyl]-methyl-amino]-cyclohexyl)-phenol,
trans-4-[4- [methyl-(2-p-tolyloxy-ethyl)-amino]-cyclohexyl]-phenol,
(RS)-4-[trans-4-(1-hydroxymethyl-3-phenyl-propylamino)-cyclohexyl]-phenol,
(RS)-4-[trans-4-(2-hydroxymethyl-3-phenyl-propylamino)-cyclohexyl]-phenol,
or
trans-N-(4-[4-[methyl-(3-phenyl-propyl)-amino]-cyclohexyl]-phenyl)-methanesulfonamide.

4. The use of a compound of formula Ib in accordance with claim 1 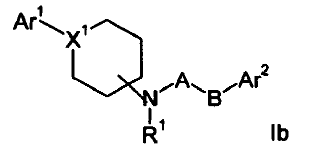 wherein Ar¹, Ar², A, B and R¹ have the significances given in claim 1 and X¹ is - C(OH)-.

5. The use of a compounds according to claim 4, which is
cis-4[1-hydroxy-4-(3-phenyl-propylamino)-cyclohexyl]-phenol.

6. The use of a compound of the formula Ic in accordance with claim 1 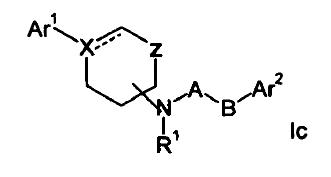 wherein Ar¹, Ar², A, B, R¹, the dotted line and X have the significances given in claim 1 and Z is -CH(CH₃)- or -C(CH₃)₂-.

7. The use of a compound according to claim 6, which is (1RS, 3RS, 4RS)-4-[3-methyl-4- [methyl-(3-phenyl-propyl)-amino]-cyclohexyl] -phenol.

8. The use of a compound of the formula Id in accordance with claim 1 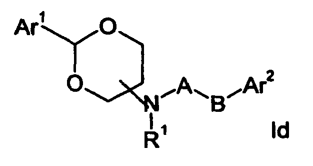 wherein Ar¹, Ar², A, B and R¹have the significances given in claim 1.

9. The use of a compound according to claim 8, which is
trans-4-[5-(3-phenyl-propylamino)-[1,3] dioxan-2-yl] -phenol.

## Patentansprüche

1. Verwendung einer Verbindung der allgemeinen Formel 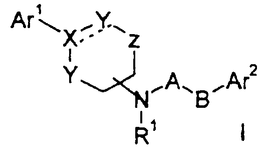 worin
Ar¹ Phenyl, Naphthyl oder Tetrahydronaphthyl, gegebenenfalls substituiert durch Hydroxy, C₁-C₄-Alkoxy, Nitro, Amino oder Methansulfonamid, ist;
Ar² Phenyl, Naphthyl oder Tetrahydronaphthyl, gegebenenfalls substituiert durch C₁-C₄-Alkyl oder Halogen, ist;
X C, CH, oder C(OH) ist;
Y -CH₂-, CH oder 0 ist;
Z -CH₂-, -CH(CH₃)- oder -C(CH₃)₂- ist;
R¹ Wasserstoff, C₁-C₄-Alkyl oder Acetyl ist;
A C=O oder -(CHR²)ₙ- ist, worin R² Wasserstoff, C₁-C₄-Alkyl oder Hydroxy-C₁-C₄-alkyl ist;
B -(CH₂)n-, 0, -CH(OH)(CH₂)ₙ-, -CH(CH₂OH)(CH₂)n-, -(CH₂)ₙCH(OH)- oder -CH(CH₂OH)- ist;
--- eine Bindung sein kann und
n 0 bis 4 ist,
und pharmazeutisch akzeptabler Säureadditionssalze davon zur Herstellung von Medikamenten für die Behandlung von Neurodegeneration, verursacht durch Schlaganfall, Hirntrauma, Alzheimer-Krankheit, Parkinson-Krankheit, Huntington-Krankheit, ALS (amyotrophe Lateralsklerose) und Neurodegeneration, die mit bakteriellen oder Vireninfektionen verbunden ist.

2. Verwendung einer Verbindung der Formel Ia nach Anspruch 1 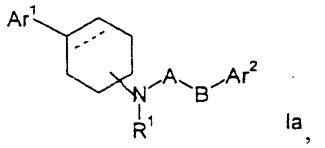 worin Ar¹, Ar², A, B, R¹ und die gepunktete Linie die gleiche Bedeutung wie in Anspruch 1 aufweisen.

3. Verwendung einer Verbindung nach Anspruch 2, die
trans-4-[4-(3-Phenyl-propylamino)-cyclohexyl]-phenol,
trans-4-[4-[Methyl-(3-phenyl-propyl)-amino]-cyclohexyl]-phenol,
trans-4-[4-[Ethyl-(3-phenyl-propyl)-amino]-cyclohexyl]-phenol,
trans-4-[4-(4-Phenyl-butylamino)-cyclohexyl]-phenol,
trans-4-[4-[3-(4-Fluor-phenyl)-propylamino]-cydohexyl]phenol,
trans-4-(4-[[3-(4-Fluor-phenyl)-propyl]-methyl-amino]-cyclohexyl)-phenol,
trans-4-[4-[Methyl-(2-p-tolyloxy-ethyl)-amino]-cyclohexyl]-phenol,
(RS)-4-[trans-4-(1-Hydroxymethyl-3-phenyl-propylamino)-cyclohexyl]-phenol,
(RS)-4-[trans-4-(2-Hydroxymethyl-3-phenyl-propylamino)-cyclohexyl]-phenol, oder
trans-N-(4-[4-[Methyl-(3-phenyl-propyl)-amino]-cyclohexyl]-phenyl)-methansulfonamid ist.

4. Verwendung einer Verbindung der Formel Ib nach Anspruch 1 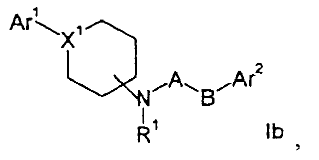 worin Ar¹, Ar², A, B und R¹ die in Anspruch 1 angegebenen Bedeutungen haben und X¹ -C(OH)- ist.

5. Verwendung einer Verbindung nach Anspruch 4, die cis-4-[1-Hydroxy-4-(3-phenyl-propylamino)-cyclohexyl]-phenol ist.

6. Verwendung einer Verbindung der Formel Ic nach Anspruch 1 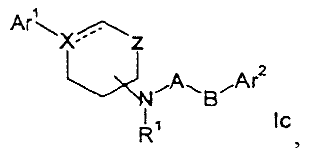 worin Ar¹, Ar², A, B, R¹, die gepunktete Linie und X die in Anspruch 1 angegebenen Bedeutungen haben und Z -CH(CH₃)- oder -C(CH₃)₂- ist.

7. Verwendung einer Verbindung nach Anspruch 6, die (1RS,3RS,4RS)=4-[3-Methyl-4-[methyl-(3-phenyl-propyl)-amino]-cyclohexyl]-phenol ist.

8. Verwendung einer Verbindung der Formel Id nach Anspruch 1 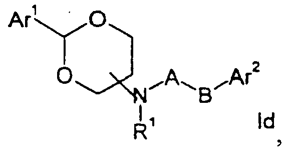 worin Ar¹,Ar²,A, B und R¹ die in Anspruch 1 angegebenen Bedeutungen haben.

9. Verwendung einer Verbindung nach Anspruch 8, die trans-4-[5-(3-Phenyl-propylamino)-[1,3]dioxan-2-yl]-phenol ist.

## Revendications

1. Utilisation d'un composé de formule générale 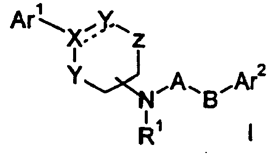 dans laquelle
Ar¹ est phényle, naphtyle ou tétrahydronaphtyle, éventuellement substitué par hydroxy, alcoxy en C₁-C₄, nitro, amino ou méthanesulfonamide;
Ar² est phényle, naphtyle ou tétrahydronaphtyle, éventuellement substitué par alkyle en C₁-C₄ ou halogène;
X est C, CH ou C(OH);
Y est -CH₂-, CH ou O;
Z est -CH₂-, -CH(CH₃)- ou -C(CH₃)₂-;
R¹ est hydrogène, alkyle en C₁-C₄ ou acétyle;
A est C=O ou -(CHR²)ₙ- où R² est hydrogène, alkyle en C₁-C₄ ou hydroxyalkyle en C₁-C₄;
B est -(CH₂)ₙ-, O, -CH(OH)(CH₂)ₙ-, -CH(CH₂OH)(CH₂)ₙ-, -(CH₂)ₙCH(OH)- ou -CH(CH₂OH)-;
--- peut être une liaison et
n est 0-4
et de ses sels d'addition d'acides pharmaceutiquement acceptables pour la fabrication de médicaments destinés au traitement de la neurodégénérescence due à une attaque, un trauma cérébral, la maladie d'Alzheimer, la maladie de Parkinson, la maladie de Huntington, la SLA (sclérose latérale amyotrophique) et la neurodégénérescence associée à des infections bactériennes ou virales.

2. Utilisation d'un composé de formule la selon la revendication 1 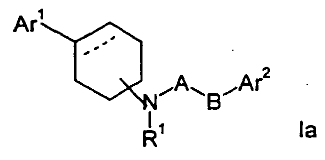 dans laquelle Ar¹, Ar², A, B, R¹ et la ligne pointillée ont la signification donnée dans la revendication 1.

3. Utilisation d'un composé selon la revendication 2, qui est
le trans-4-[4-(3-phénylpropylamino)cyclohexyl]phénol,
le trans-4-[4-[méthyl-(3-phénylpropyl)amino]cyclohexyl]phénol,
le trans-4-[4-[éthyl-(3-phénylpropyl)amino]cyclohexyl]phénol,
le trans-4-[4-(4-phénylbutylamino)cyclohexyl]phénol,
le trans-4-[4-[3-(4-fluorophényl)propylamino]cyclohexyl]phénol,
le trans-4-(4-[[3-(4-fluorophényl)propyl]-méthylamino]cyclohexyl)phénol,
le trans-4-[4-[méthyl-(2-p-tolyloxyéthyl)amino]cyclohexyl]phénol,
le (RS)-4-[trans-4-(1-hydroxyméthyl-3-phénylpropylamino)cyclohexyl]phénol,
le (RS)-4-[trans-4-(2-hydroxyméthyl-3-phénylpropylamino)cyclohexyl]phénol,
ou le trans-N-(4-[4-[méthyl-(3-phénylpropyl)amino]cyclohexyl]phényl)méthanesulfonamide.

4. Utilisation d'un composé de formule Ib selon la revendication 1 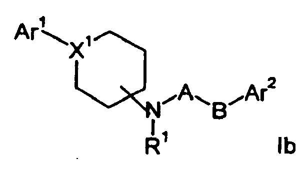 dans laquelle Ar¹, Ar², A, B et R¹ ont la signification donnée dans la revendication 1 et X¹ est-C(OH)-.

5. Utilisation d'un composé selon la revendication 4, qui est le cis-4-[1-hydroxy-4-(3-phénylpropylamino)cyclohexyl]phénol.

6. Utilisation d'un composé de formule Ic selon la revendication 1 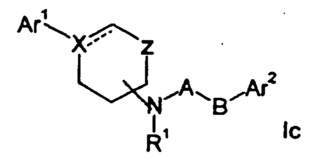 dans laquelle Ar¹, Ar², A, B, R¹, la ligne pointillée et X ont la signification donnée dans la revendication 1 et Z est -CH(CH₃)- ou -C(CH₃)₂-.

7. Utilisation d'un composé selon la revendication 6, qui est le (1RS, 3RS, 4RS)-4-[3-méthyl-4-[méthyl-(3-phénylpropyl)amino]cyclohexyl]phénol.

8. Utilisation d'un composé de formule Id selon la revendication 1 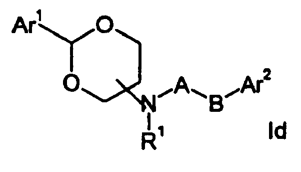 dans laquelle Ar¹, Ar², A, B et R¹ ont la signification donnée dans la revendication 1.

9. Utilisation d'un composé selon la revendication 8, qui est le trans-4-[5-(3-phénylpropylamino)-[1,3]dioxan-2-yl]phénol.
